Europäisches Patentamt

European Patent Office

Office européen des brevets

(11) Publication number: **0 661 647 A1**

# EUROPEAN PATENT APPLICATION

(21) Application number: 93403215.2

(51) Int. Cl.6: **G06F 17/00**

(22) Date of filing: **31.12.93**

(43) Date of publication of application:
**05.07.95 Bulletin 95/27**

(84) Designated Contracting States:
**AT BE CH DE DK ES FR GB GR IE IT LI LU MC NL PT SE**

(71) Applicant: **INSTITUT PASTEUR**
**25/28, rue du Docteur Roux**
**F-75015 Paris (FR)**

(72) Inventor: **Yeramian,Edouard**
**50 rue des Martyrs**
**F-75009 Paris (FR)**

(74) Representative: **Martin, Jean-Jacques et al**
**Cabinet REGIMBEAU**
**26, Avenue Kléber**
**F-75116 Paris (FR)**

(54) **A method and system for modelizing partition functions of sequences.**

(57) A method for modelling a partition function in a sequence of elements having a length $\underline{n}$, said partition function taking into account N mutually coupled long-range effects, each element being capable of taking either of a discrete number of accessible states, in particular two respectively open and closed states for the case of a macromolecular sequence, said partition function Z(n) being equal to the sum of n individual terms C(i) each corresponding to a partial contribution to the total partition and iteratively calculated along the sequence, each term being equal to the sum of the statistical weights at each element of the sequence for all the possible configurations of said sequence, said method being implemented in a computer having a processor and a memory and being characterized in that it includes the following steps :

(a) representing each long-range effect in the form of a sum of exponential terms,

(b) storing into said memory an initial smooth propagator in the form of a N-dimension matrix of cells, each cell of said smooth propagator being representative of the merging of successive terms $O_{...,k,j}(i)$ corresponding to irreducible configurational classes of said possible configurations;

(c) successively merging the corresponding irreducible configurational classes into the corresponding cells of said smooth propagator by means of the exponential terms of said long range effects;

(d) repeating step (c) for all sub-sequences of the sequence, whereby all terms C(1) to C(n) of the partition function are obtained.

The present invention concerns a general procedure which represents the only possible rigorous solution for making tractable various classes of sequence-dependent calculations.

The models considered are characterized by the following basic features:

### 1) Primary sequences:

Sequences are meant in a wide sense, but biological sequences represent the main object of interest for the procedure presented in the Patent. Following the molecules considered (DNA, RNA, peptides, etc), the primary sequences are expressed with use of the corresponding appropriate alphabets (letters "A", "T", "G" and "C" for DNA molecules, for example). For structural problems we consider as a starting point a primary sequence of length n. For problems such as those concerned with sequence comparisons or the search for homologies, several sequences of lengthes n, m, etc, are considered simultaneously.

### 2) Specification of a discrete number of possible states for each element in a sequence:

A discrete number of possible "states" is supposed to be accessible to each element (a base pair for a DNA sequence, etc) at position i of a given sequence.

The word "state" is also meant in a wide sense. In structural models we may consider configurational states. For example in models of helix-coil transitions in DNA molecules (Ref. 1) a base pair at position i is either in the coiled state (represented with the symbol 0) or the helical state (represented with the symbol 1). Similarly, in structural models for peptides, an amino acid can be in the coiled state, the helical state, participate to a beta-sheet structure, etc. In spatial conformational (geometric) descriptions, the discrete states can represent angular parameters such as those used classicaly in the "Rotational Isomeric State Approximation" (see, for example, Ref. 2).

In sequence comparisons or the search for homologies, a "state" can be an abstract entity, representing for example through a binary symbol (0 or 1, exactly as for the helix-coil model above) the occurrence of a homology for given relative positions of two (or more) sequences, and given inserts and gaps.

### 3) Specification of an "interaction" model, for the weighting of each particular configuration:

With the specification of the sequence(s) and the various accessible states for each element, various problems of interest rely on the specification of a model of "interactions". The word "interactions" is also meant in a wide sense: each element at a given position and in a given state may interact with any other element at any other position and also in any given state.

The simplest models of interactions may be considered in which only neighbour elements are supposed to interact. Such simple interactions are not of real interest in many cases, as realistic models make it necessary to consider, on the contrary, long-range interactions, through which parts which are remote on the primary sequence are made to interact in some given sense.

In configurational models such interactions can account for effective contacts between elements which are remote on the primary sequence. An archetypal -simple-model for long-range interactions is provided by the classical model of helix-coil transitions in DNA molecules, in which length-dependent weights $\omega(j)$ must be attributed to j contiguous elements in the coiled state, with elements in the helical state at both extremities of the coiled regions.

Similarly, in treatments concerned with sequence comparisons and the search for homologies, in a more formal sense, we need to consider "interactions" which extend beyond nearest-neighbours, for example for the attribution of a length-dependent weight $\omega(j)$, when a "gap" of length j is introduced in a particular configuration concerning the relative positions of the two sequences which are to be compared.

For a given model, with the specification of the possible interactions, a "weight" can be attributed to each configuration. For a model involving a sequence of length n, and with k possible discrete states for each element of the sequence, there are $k^n$ possible configurations.

Beyond the various long-range interactions, the sequence-specificty is also involved in the weighting scheme. For example in models of helix-coil transitions, a weight $s_i$ is attributed to an element at position i, in the helical state, following it's chemical composition ($s_{AT}$, respectively $s_{GC}$, if the base pair at position i is of the A-T type or respectively the G-C type). Such sequence-dependent parameters may themselves depend on any set of external parameters which describe the system, such as temperature, etc.

For various problems of interest, a given quantity characterizing the system under study must be evaluated. For example, in configurational models, we may be interested in evaluating the probability for an element at position i to be in a given state, or the fraction of elements which are in a specified state, under specified external conditions (typically in the classical helix-coil transition model we may evaluate the fraction of base pairs which are in the coiled state at a given temperature).

For such evaluations we need to consider the combinatorial description underlying the $k^n$ various possible configurations, with the proper weight -following the adopted model- attributed to each particular configuration. The sum of the corresponding $k^n$ weights is classically known as the partition function associated with the model. It is the evaluation of such partition functions which represents the major technical difficulty in many cases. From the partition function, various quantities of interest attached to a given model are then readily evaluated.

An appropriate "measure" for the technical difficulty in the evaluation of a given model is provided by the algorithmic complexities of the model. A model is said to be in time -respectively space- complexity in $O(g(n))$ (for a sequence of length n) if the time requested for it's calculation -respectively the computer memory storage requirements-grow proportionally to g(n), with n.

Because of the sequence-specificity we consider treatments which are processive along a given sequence. Techniques relevant to Dynamic Programming can be used for such processive evaluations of partition functions.

Relative to the procedure introduced in the Patent, the fundamental point is that for many models of interest, prohibitive calculation times are not attributable to the huge number $k^n$ of possible configurations per se, but only to the necessity of handling various long-range effects in the models.

This fact is strikingly illustrated by the protein folding problem as formulated in Levinthal's paradox: the time required for exploring the configurational space associated with $3^{100}$ possible configurations (for a chain of length 100 and with 3 possible states for each element) would be longer that the age of the universe. Such a requirement is entirely attributable to the long-range effects which must be treated in the model, as for a nearest-neighbour model involving exactly the same total number of configurations, the calculations are performed in negligible time.

For a wide range of models in the field described above, the procedure according to this invention represents the only possible solution for treating rigorously sequence-dependent models involving mutually coupled long-range effects, with algorithmic time complexities relevant to the simplest nearest-neighbour models. The algorithmic time complexities of the models are typically of the form $O(n^k)$, whereas the algorithmic complexitiy for a nearest-neighbour treatment is of the form $O(n)$. Such a reduction in the algorithmic complexities can be associated with very significant accelerations in the calculations. In typical cases, calculations may be accelerated by factors of the order of 100 million times.

The procedure relies on the processive handling of the various long-range informations in the model through the definition of an operator which is called "Smooth-Propagator" in the Patent (see below). In this operator the various long-range effects are represented as multiexponential functions.

The procedure is very general in it's essence. By construction the procedure is illustrated in detail through appropriate model-problems. The Patent concerns nevertheless specifically the generality features in the procedure, which can be formulated through many variants and adaptations, following the particular models treated.

A general "historical" background is presented for the procedure, highlighting the choice of the model-problems for the illustration of the general new principle. A detailed presentation of the various fundamental steps involved in the procedure is then presented, as illustrated through the treatment of the chosen model-problems. A number of variants, adaptations and of the procedure are then presented. This enumeration is by no means exhaustive with respect to all the potential derivations and generalizations based on the procedure, as presented here.

## Background:

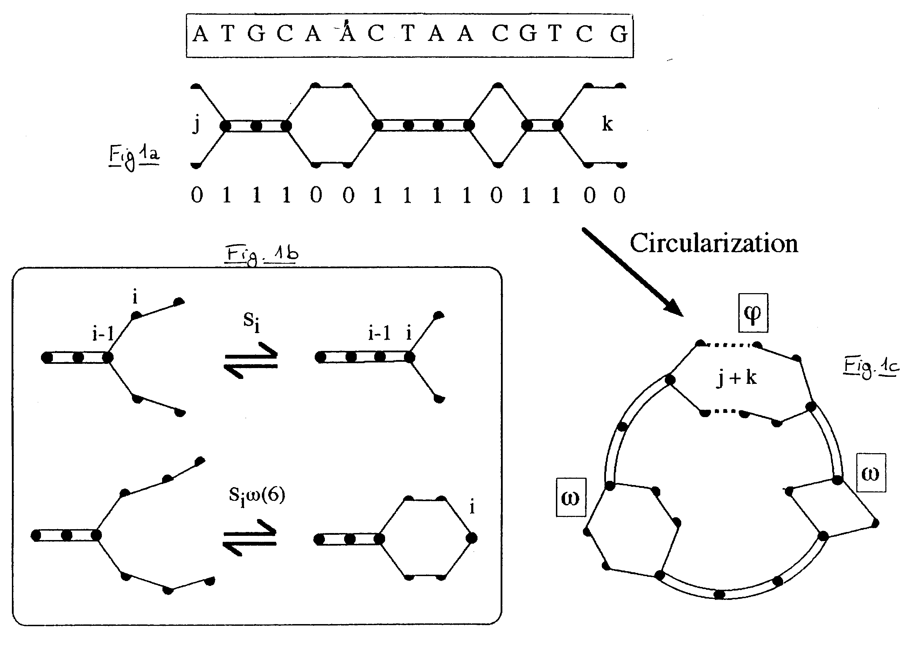

The physico-chemical bases for the theory, in statistical mechanics, of sequence-dependent helix-coil transitions are recalled in Fig. 1. We consider the introduction of the constraint of circularity in the classical, linear, model with the following rationale:

-The linear model is a textbook classics (Ref.1), associated with the foundations of structural molecular biology, and the theoretical treatment of this model is, "historically", at the basis of methodological developments for sequence-dependent problems. The loop-entropy effect $\omega$ in this model (see Fig.1) stands as an archetype for long-range effects -extending throughout the length of the molecule.

-The intrinsic time complexity of the linear model is $O(n^2)$ (see below). This model represents the only case for which an appropriate rigorous solution, with reduced algorithmic complexity, was developped. The corresponding solution represents the culmination of a "quest" towards a rigorous and tractable formulation, extending over a period of about twenty years (1959-1977), with the three following important landmarks:

1) For a sequence of size n, a direct application of the generic transfer matrix method developed in physics, essentially for homogeneous systems, was first expressed (Ref.3) as the product of n matrices of size $2^{n-1}x2^{n-1}$. Removing redundancies in the handling of the information in this scheme, Litan (Ref.4) devised a drastic reduction in the size of the matrices to nxn. Ever since that achievement in 1968, it was consistently reported by the workers in the field that the corresponding Simplified Matrix Method is in time complexity $O(n^3)$.

2) For the exact treatment of the linear model, it was alleged accordingly that the above, generic, framework did not allow an optimal reduction of the time complexity to the intrinsic complexity of the model, which was achieved only at the price of a bifurcation towards more specialized formulations. In this logics -giving up the evaluation of the partition function- the Poland's algorithm (1974) was developped as an exact scheme in $O(n^2)$ based on recurrence relations specific to the conditional probabililies of the model (Ref.5).

3) By generalizing an idea initially proposed by Frank-Kamenetskii and Frank-Kamenetskii and adapting it in the framework of Poland's algorithm, Fixman and Freire developed an algorithm in time complexity $O(n)$ (the Poland-Fixman-Freire (Ref.6) algorithm, "PFF", 1977) based on the numerical expression of the long-range effect as a multiexponential function. This treatment is not exact but it is rigorous as it introduces no approximations in the basic physics of the model.

-No extensions -in any field- were derived to this formulation for complexity build-up, involving mutually coupled long-range effects. Increasingly more complex models could be handled only at the price of more or less drastic simplifications in the physics of the interactions (as a clearcut distinction from the numerical approximation involved in the rigorous PFF). The plausible explanation for the lack of generalizations to the concepts in the PFF is it's derivation in the prolongation of specialized formulations, following the methodological bifurcation referred to above. In such a context, the status of the prototype-solution was that of a clever "numerical trick", the scope of which is limited to the specific model.

-Following the above "historical" background, complexity build-up starting from the linear helix-coil model as a template, represents the ideal "benchmark" for adressing the methodological problem of the existence of prototype-solutions for increasingly more complex models, involving mutually coupled effects. The circularization of this template, as represented in Fig.1, represents the simplest scheme involving (two) mutually coupled long-range effects: the long-range effect attached to loops resulting from the joining together of free-ends of the linear configurations ("labelled" $\phi$, see Fig.1) is characteristic of a long-range effect coupled with $\omega$, as treated in the linear model. Through the introduction of this constraint, the time and space complexities of the model shift one order of magnitude, as compared to the linear model, respectively from $O(n^2)$ to $O(n^3)$, and from $O(n)$ to $O(n^2)$ (see below).

-For the linear problem, the SIMM (Simplified Itertative Matrix Method, see Ref. 7) was developed as an exact scheme with time complexity $O(n^2)$, within the generic framework referred to above, based on the configurational description of the problem and the explicit evaluation of the partition function (from which various properties, such as conditional probabilities can be readily calculated). In the light of this formulation, it appears that the bifurcation towards specialized treatments -as discussed above- could be attributed to a too rigid conceptual association of the Simplified Matrix Method with it's origin in physics for homogeneous systems (eigenvalue calculations for appropriate matrices, see Ref.7 for a more detailed discussion).

Based on the expression of the long-range effect $\omega$ as a sum of exponentials, the F__SIMM (Ref.7) was derived, in the prolongation of this scheme, as a rigorous formulation in $O(n)$. For the linear model, with respect to efficiency criteria, this treatment does no better than the prototype-solution PFF as described above; it demonstrates, nevertheless, that such efficiency can be obtained without the need for bifurcations towards specialized formulations. This feature is put here to advantage for the derivation of methodological solutions for complexity build-up in the models.

### Theoriesof Helix−coil transitions in DNA molecules (see Fig.1):

In the classical model of helix-coil transitions in DNA molecules, it is supposed that there are two accessible states for each base pair at position i in a sequence of length n: the closed state (hydogen-bonded) represented with the symbol 1 and the open state (breaking of the hydrogen bonds) represented with the symbol 0. For a linear DNA, with a hypothetical sequence, one such particular configuration, is represented schematically in a, with helical regions ("ladders") and open regions (single-stranded "loops" at the interior of the molecule or coiled free-ends).

4

The problem in statistical mechanics is the evaluation of the so-called partition function associated with the model, from which any quantity of interest can be calculated straightforwardly. The partition function is obtained as the sum, over the $2^n$ possible configurations, of the statistical weights attributed to the various configurations. The statistical weight of a given configuration is the equilibrium constant for it's formation, expressed as a product of the elementary equilibrium constants (represented schematically in b) for each element at position i in the sequence. The various weights are defined relative to a ground-state energy corresponding to the unconstrained single-strands, and accordingly the weight 1 is attributed to open regions occuring at the ends of linear molecules. A statistical weight $s_i$ (depending on the base pair A-T or G-C at position i) is attributed for the "stacking" of the closed base pair at position i on a base pair already in the helical state at position i-1. On the other hand, a closed base pair at position i following a base pair in the open state at position i-1, corresponds to the initiation of a helical region, the physical representation of which depends on the particular model adopted. In the nearest-neighbour model, $s_i$ is multiplied by a constant (corrective) factor $\beta$. More realistically, the long-range constraint introduced by the closure of a single-stranded ring with k open base pairs ($2k+2$ elementary single-stranded internucleotide links) is accounted for by the attribution of a length-dependant statistical weight $f(k) = \omega(2k+2)$. The "loop-entropy function" $\omega$, reflects the probability of closure of a single-stranded ring, associated with an important decrease in the degrees of freedom compared to a free-coil. It can be evaluated in polymer physics (Ref. 8,9) or measured experimentally, under various conditions.

The introduction of the formal constraint of circularity in a DNA molecule is illustrated in c for the linear configuration in a: the free-ends with respectively j and k open base pairs are fused together leading to a single loop of length j+k open base pairs for the circular configuration. On physical grounds this effect is identical to $\omega$, but, on methodological grounds, the correlations associated with circularity strictly correspond to the introduction in the model of a second long-range effect -labelled $\phi$- coupled with $\omega$. With this respect, the fundamental methodological problem in complexity build-up is not the number -per se- of long-range effects (with either identical or different physical significations), but the occurence of couplings between various effects. For example, a generalized linear model can be elaborated, in which, along with the long-range effect $\omega$, a long-range effect $\Phi$ is attached to helical regions (accounting, for example, for electrostatic effects and global dipole moments, see Refs. 10 and 11). The two, physically distinct, effects $\omega$ and $\Phi$ in this generalized model do not lead to a scheme of coupled effects, as a difference from the circular model with the effects $\omega$ and $\phi$. Accordingly, the time complexity of the generalized linear model is the same than that of the "ordinary" linear model, whereas a shift one order of magnitude occurs for the circular model, as demonstrated below.

The circular problem is treated here in a molecule supposed to be topologically unconstrained. We refer to this model as the "formal" circular model. *This formal circular model represents an appropriate model−problem for the detailed description of procedure SIMEX, in this Patent, for the handling of increasingly more complex models involving mutually coupled long−range effects.*

One particular example for the general applicability of the procedure concerns the introduction of the topological constraint of supercoiling, in this formal circular model. Following presently accepted theories, the topological constraint of supercoiling is a global effect modulating helix-coil transitions via a global parameter which takes into account only the *total* number of open base pairs in the molecule (Refs. 12, 13 and 14).

**Detailed description of the basic steps involved in the general procedure of this invention, as illustrated through the treatment of appropriate model problems:**

In what follows the various steps are illustrated with the model-problems of helix-coil transitions in DNA molecules.

# First step: Processive formulation of the exact treatments, determination of the intrinsic algorithmic complexities of the models.

In helix coil-transition theories, the statistical mechanics problem is the evaluation of the partition function $Z^{(n)}$ -for a sequence of length n- which is the sum of the statistical weights associated with the $2^n$ possible configurations.

We consider strictly processive treatments along the sequence. For the sequence-specific problem, the evaluation of the partition function splits into the evaluation of n terms C(i) ($1 \leq i \leq n$): C(i) is the sum of the

5

statistical weights associated with configurations characterized by a base pair at position i in the closed state, and base pairs from i + 1 to n in the open stale, with $Z^{(n)}$ the sum of the C(i)'s.

At step i, the calculations are performed relative to the subsequence of length i (from 1 to i). In linear models, C(i) calculated for this subsequence coincides with the value of C(i), as defined above, for the whole sequence, because open regions at the extremities of the molecule correspond to single stranded free-ends, with the weight one, following the definition of the ground-state (see Fig.1). For the circular model, the sequence-specificity makes it necessary to devise a processive treatment along a linearized molecule, with an arbitrary cut specifying the position 1. In this case, the evaluation of C(i) for the whole molecule, involves the handling of the detailed informations concerning the "free-ends" of the linearized molecule, which must be fused into single loops for the circular configurations, with the proper attribution of the corresponding long-range weights, $\phi$.

### *Grouping together of configurations into Irreducible Configurational Classes (ICC's):*

For a given model, reduction in algorithmic complexities can be achieved -as compared to the complexity $O(2^n)$ associated with an exhaustive enumeration of the configurations- if, in the calculations, various configurations can be grouped together into appropriate classes. If through such a grouping together, the processive evaluation of the C(i)'s involves the handling of only the strictly necessary information -with no redundancies- we call the corresponding classes, Irreducible Configurational Classes (ICC's) of the model.

### *Definition of the exact processive treatments:*

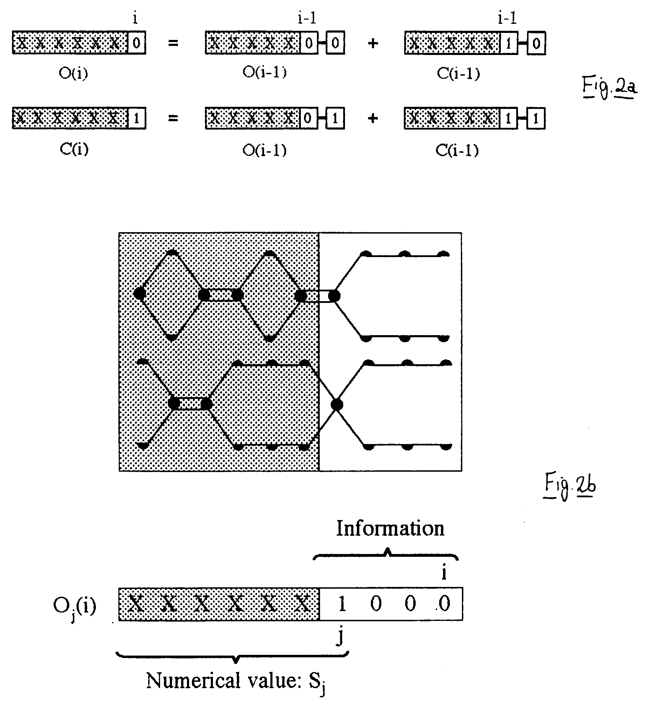

We consider first the nearest-neighbour model. At step i, for the subsequence of length i, the partition function -$Z^{(i)}$- is the sum of two contributions: $Z^{(i)} = C(i) + O(i)$, with O(i) the sum of the weights of the configurations ending with a base pair in the open state at position i. By definition, C(i) can be evaluated if and only if O(i-1) and C(i-1) are known, the evaluation, at step i, of C(i) then simply writes (Fig.2a):

$$C(i) = O(i-1)oc(i) + C(i-1)cc(i), \qquad [1]$$

with oc(i) and cc(i) designating, respectively, the sequence-dependent- statistical weights attributed for the base pair at position i in the closed state (c), depending on the state of the neighbour element at position i-1 (o for open and c for closed). An analogous relation holds for O(i) (Fig.2a). C(i) and O(i) are the ICC's of the model, and the scheme is in space complexity O(1), and time complexity O(n).

As illustrated by the simple treatment above, the derivation of a processive formulation, of minimal algorithmic complexities, relies on the concomitant utilisation of two fundamental -corrected- features in the definition of the ICC's of a model:

1) An ICC is defined by an *informational content* , the configurational description common to the various configurations grouped together, of which it allows precisely to keep track. Beyond the ICC's, any further grouping together of the configurations impedes the evaluation at step i of C(i) (as would be the case in the model above if O(i-1) and C(i-1) were merged at step i-1). Accordingly, through the definition of the ICC's, the "book-keeping" of the information is reduced to the strict minimum and the space complexity of the scheme is minimal.

2) The numerical value of an ICC, at a given step i, is the *sum* of the statistical weights (for the subsequence of length i) of the configurations grouped together. By virtue of the corresponding *numerical factoring* , the time complexity of the scheme is minimal: the elongation of various configurations in an ICC, with the element at position i + 1, in a given state, is performed with a single multiplication. This numerical feature is intricately connected with the informational feature above, as the weight attribution for the elongation step is dictated by the informational content of the ICC. In what follows, the generic symbol S (with appropriate indexes) will be used for the numerical values of the ICC's whenever an explicit distinction from their informational representations will be desired.

With increasingly more complex models -as a mere extension of the simple scheme above- the onset of untractability is reached because of the increasingly more tedious task involved in the handling and book-keeping of increasingly more detailed informations.

For the linear model, the informational content of O(i), as defined above for the nearest-neighbour model, is no more sufficient. For the proper handling of the long-range effect in the model, we need to keep track of the information concerning the number of contiguous open base pairs at the right-ends of the configurations, because, by definition, as represented schematically in Fig.2b, it is precisely this information which is converted -upon ring-closures- to numerical contributions in the form of length-dependent loop-entropy weights. Following this constraint, O(i) must be subdivided into the i-1 ICC's $O_j(i)$, as represented in Fig.2c, specifying the lengthes of the right-end open regions (from j + 1 to i). At each step i, there is one additional ICC for configurations ending with an open base pair ($O_{i-1}(i) = C(i-1)$, the equality holds between the numerical value of an ICC at step i-1 and that of an ICC at step i). With the stepwise increase of the total number of ICC's, along the processive treatment, the space complexity of the scheme is in O(n).The evaluation of C(i), is based on the ring-closures corresponding to the i-2 $O_j(i-1)$'s. As each ring-closure represents a single multiplication, C(i) is calculated as a scalar product (Fig.2c) the size of which increases stepwise, leading for the overall scheme to a time complexity in $O(n^2)$.

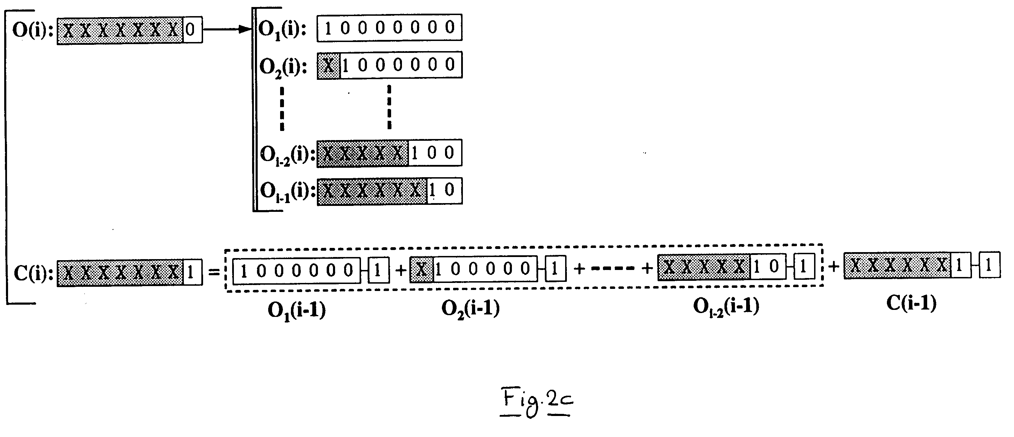

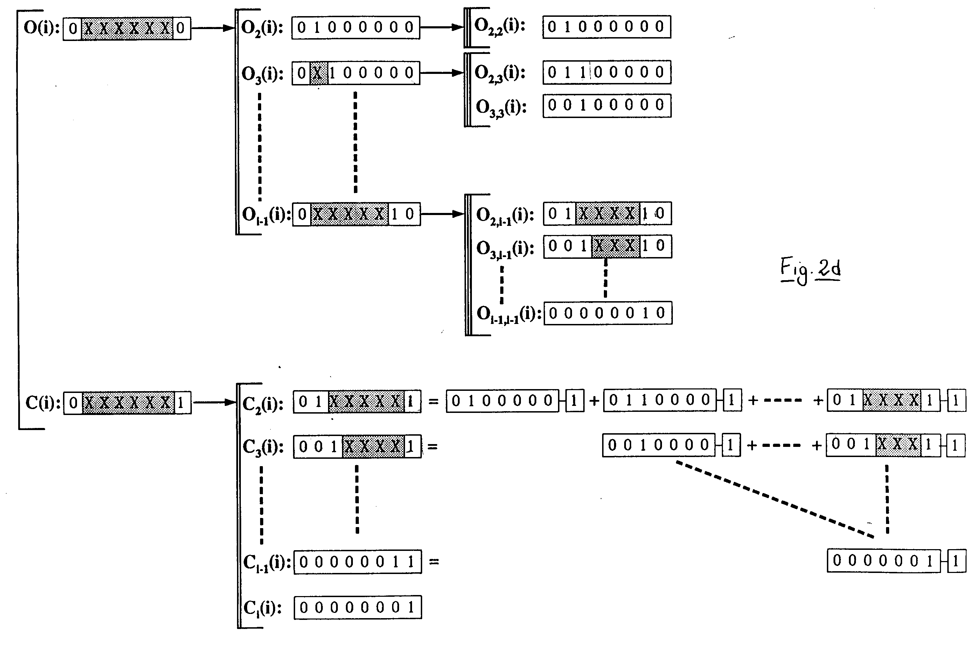

For the circular model, with exactly the same rationale, in order to ensure the ring-closures (with the weights labelled $\phi$) resulting from the joining together of the two free-ends of the linearized molecule, we must, in addition, keep track of the detailed informations concerning the left-end open regions. Accordingly, the class C(i) of the circular model subdivides into the i ICC's $C_k(i)$ ($1 \leq k \leq i$), based on the specification of the lengthes (k-1) of the left-end open regions ($C_1(i)$ is associated with configurations with a closed base pair at position 1). The processive evaluation of the $C_k(i)$'s, involving right-end ring-closures, makes it necessary -for the internal coherency of the scheme- to specify the same detailed left-end informations for the class of configurations characterized by open base pairs at the right-ends. As illustrated in Fig 2.d, this requirement leads to the introduction, in this generic class O(i), of a third-order subdivision, corresponding to the specification of the left-end informations for the $O_j(i)$ as defined above. The resulting $O_{k,j}(i)$'s (open base pairs from 1 to k-1, and from j + 1 to i) -along with the $C_k(i)$'s- are the ICC's of the circular model. With this proper book-keeping of the right-end and left-end informations, the calculation of each $C_k(i)$ is equivalent to a whole linear problem of size i-k + 1. Associated with this third-order subdivision, the time and space complexities of the model are respectively in $O(n^3)$ and $O(n^2)$.

Generalizing the above rationale, complexity build-up in the models proceeds through a *nested hierarchy* of successive subdivisions for the definition of the corresponding ICC's, synonymous with intractability in practice.

**Second step : formulation of the procedure allowing to perform the same calculations with reduced algorithmic complexities.**

We consider first the linear model and we want to treat it with algorithmic complexities reduced to that of the nearest-neighbour model, respecting the rigorous representation of the underlying physics.

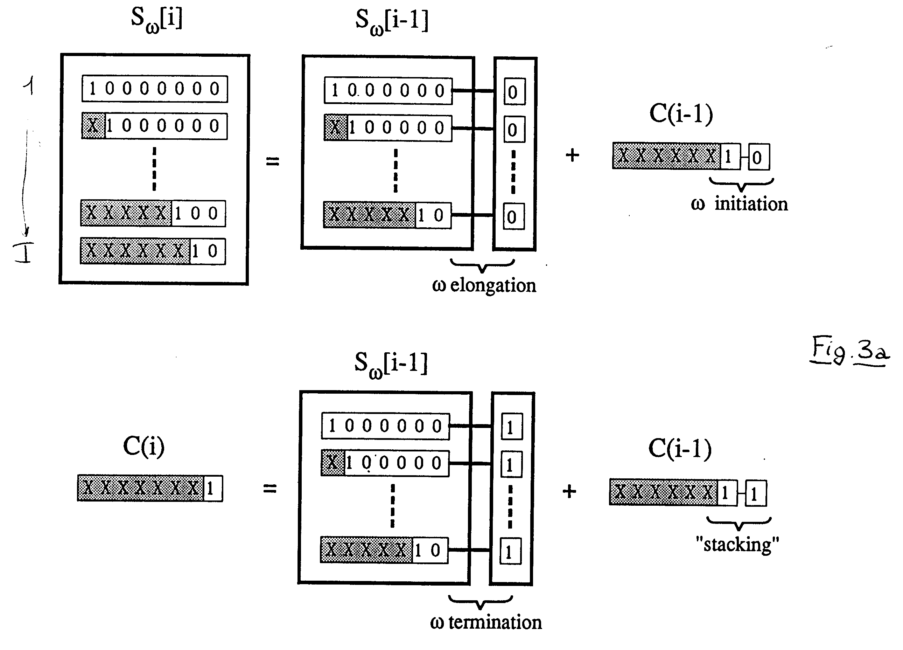

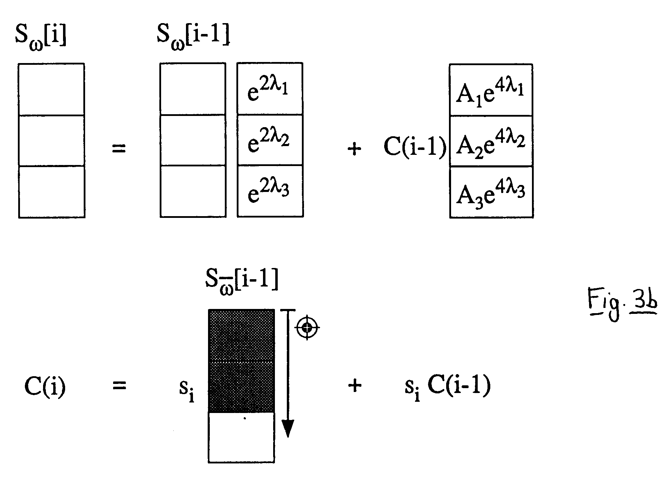

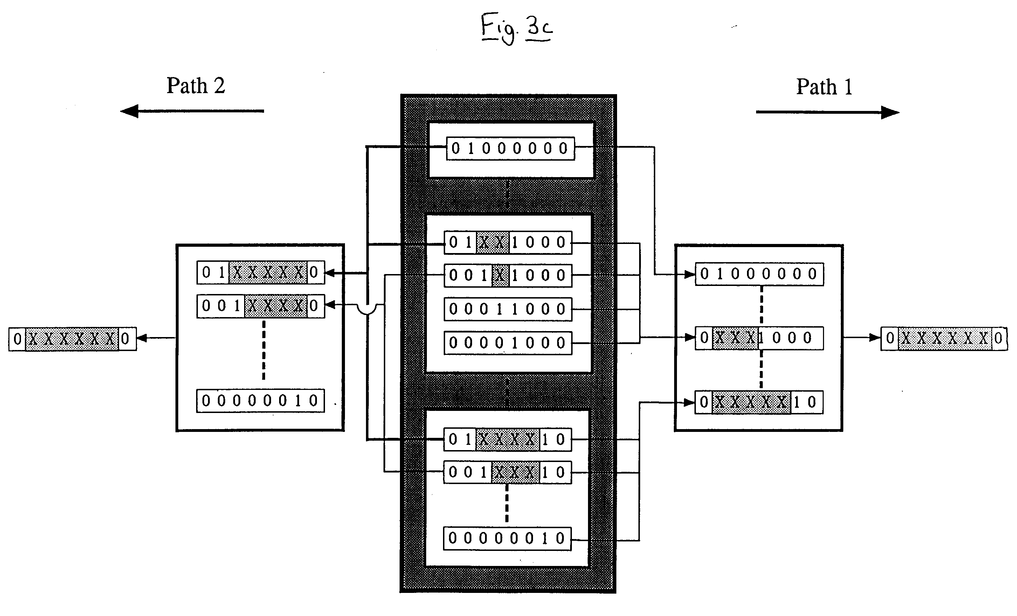

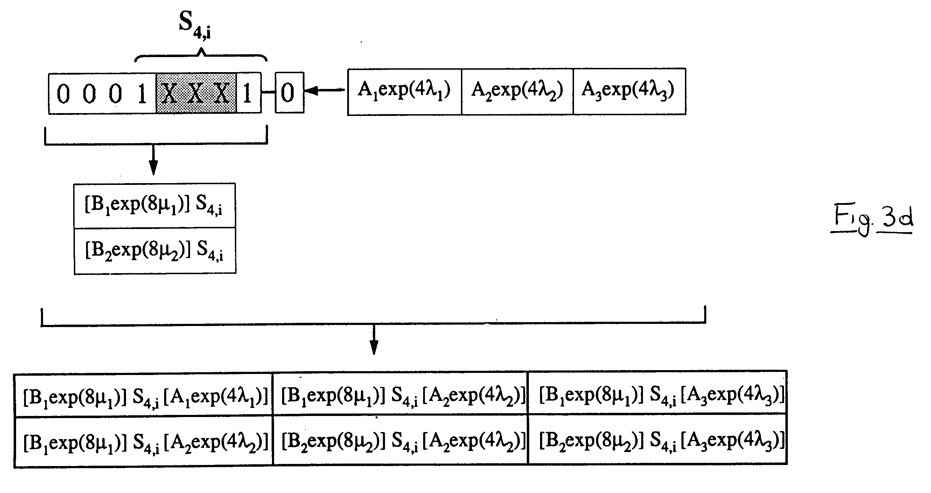

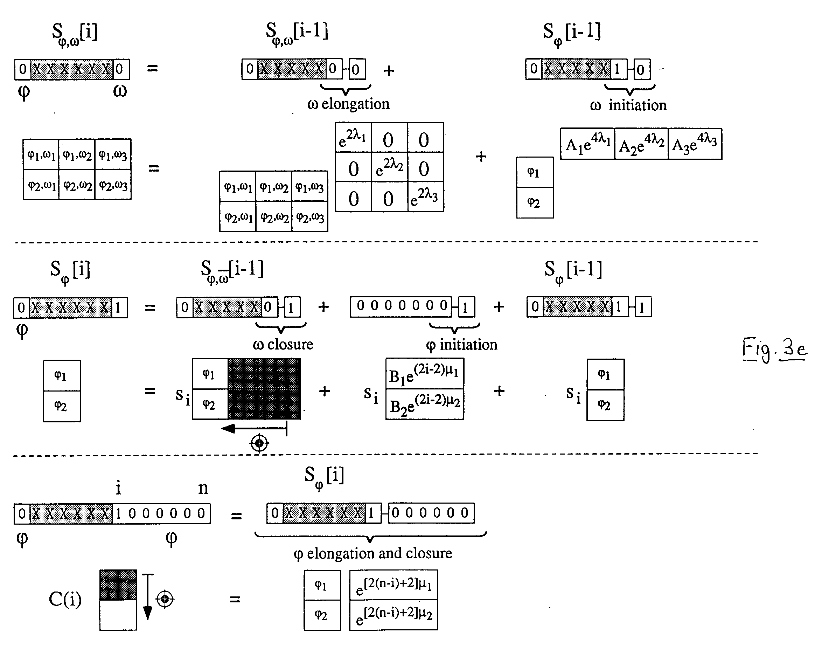

Any grouping together of the configurations beyond the ICC's of the exact treatment above, is illegitimate with respect to the informational integrity of the model. Nevertheless, for our purpose, we have no other choice than to cancel out the subdivision of O(i) into the i-1 irreducible classes $O_j(i)$'s. As represented with the cartoon in Fig 3.a, such a "reverse" scheme, amounts in merging the ICC's $O_j(i)$ of the model in a single "box", standing for the nearest-neighbour class O(i). With this representation, the formulation of the linear model is made to resemble to the nearest-neighbour treatment (Fig.2a), and the methodological problem is to convert this wishful thinking into an operational algorithm.

*One lone−range effect: merging of the ICC's in a one−dimensional Smooth−Propagator.*

The merging operation corresponds to the summation of the ICC's, with the expected benefit of a numerical factoring, synoymous with one order of magnitude reduction in algorithmic complexities. However, such a summation cannot be performed directly, because the informational contents of the ICC's would be irremediably lost in the operation. As compared to the exact scheme above, it is necessary to revise completely the logics for the handling of the information. It will be no more possible to postpone the conversion of the informational content of each ICC ($O_j(i)$) to it's appropriate numerical representation (long-range statistical weight $\omega$) to the -final- stage of ring-closures. Instead, following the nearest-neighbour logics, a "slicing" must be introduced in this conversion, with partial contributions added *locally* to the merged ICC's, as a whole. For the consistency of the scheme it will be required that various local contributions add up properly, allowing the *global* reconstitution of the long-range effects, attached to each one of the merged ICC's.

Following the above constraint, the numerical value, $S_j$ ( $= C(j)$), of an ICC $O_j(i)$ as calculated in the exact treatment, must be completed with a numerical representation f(i-j) for it's informational content (the length i-j of the right-end open region) required to act as numerical "handle", for subsequently passing on partial contributions to the long-range effect, added locally. If such a handle exists, it must be attached to an ICC before merging it with any others, and the fundamental nearest-neighbour elongation condition for the merged ICC's then writes :

$$[S_1 f(i-1) \; + \; S_2 f(i-2) + ... + \; S_{i-1} f(1)] f(1) \; = \; S_1 f(i) \; + \; S_2 f(i-1) + ... + \; S_{i-1} f(2) \qquad [2]$$

This equation leads to the generic condition: $f(x)f(y) = f(x+y)$, for which there exists one, and only one, solution in the form of the exponential function. Requiring for the long-range effect $\omega$ to be an exponential function would be very restrictive with respect to the physics of the problem. Invoking classical concepts in numerical analysis - such as Fourier approximation- it is natural to think to sums of exponentials for overcoming this restriction. Resorting to appropriate methods in signal analysis, it should be possible with multiexponential functions to provide a representation of the long-range effect with the desired accuracy. Unfortunately, the fundamental property characterizing a single exponential is no more valid for a sum of such terms. Finally, for getting the best of both worlds, combining the fundamental property with the numerical accuracy, the solution is 1) to express $\omega$ as the sum of I exponentials:

$$\omega_{exp}(j) = \sum_{k=1}^{I} A_k \exp(\lambda_k j) \qquad [3]$$

and 2) to split the calculations into I separate terms, preserving the fundamental property following each exponential component and the associated slicing in the handling of the long-range effect.

Following this concept, the natural mathematical structure for the box represented diagramatically in Fig.3a, enclosing the merged ICC's, is a vector with I cells, we call the one-dimensional smooth-propagator $S_\omega[i]$ of the scheme. The kth cell, $S_{\omega(k)}[i]$, of this vector is devoted to the calculations following the kth exponential component, with the value:

$$S_{\omega(k)}[i] \; = \; S_1 A_k \exp((2i-2)\lambda_k) \; + \; ... + \; S_{i-1} A_k \exp(4\lambda_k) \qquad [4]$$

reflecting exactly the merging scheme as discussed above, with the handles relative to the kth exponential component.

### *Implementation of the initiation, elongation and termination steps for the one−dimensional smooth−propagator:*

As a direct result of this structure of $S_\omega[i]$, the overall consistency of the scheme, as detailed in Fig.3b, relies on the proper implementation, at a given step i, of the following three fundamental operations concerning the "sliced" handling of the long-range effect:

1) The *initiation* operation, in which the kth exponential handle ($A_k \exp(4\lambda_k)$) is attached to the ICC $O_{i-1}$-(i) (= C(i-1), with a single open base pair at the right-end, allowing it's merging with the other ICC's $O_j(i)$ in $S_{\omega(k)}[i]$.

2) The *elongation* step, in which the local contribution to the long-range effect associated with one open base pair, at position i, is added to each one of the merged ICC's. By virtue of the numerical factoring, for the kth cell of the smooth-propagator, this elongation is performed as a single multiplication (without the prefactor $A_k$, for the kth exponential component), the expression of which is: $S_{\omega(k)}[i-1]\exp(2\lambda_k)$.

3) The *termination* step, associated with ring-closures, in which the numerical representations for the long-range effects attached to the various ICC's, are "reconstituted", through their multiexponential expressions, by adding the I cells of $S_\omega[i]$.

### *Hierarchical merging of Smooth−Propagators of increasing dimensionalities for the treatment of models involving mutually coupled long−range effects.*

The present invention directed to this aspect, is defined in the appended claims.

For complexity build-up, the concepts at the basis of the above scheme, must be generalized for the handling of mutually coupled long-range effects, as illustrated with the circular problem. We concentrate here on the configurations with an open base pair at position 1 (the only ones relevant to the problem of coupled effects, see Fig.2), and various C(i)'s and O(i)'s refer only to that category.

At a given step i, the evaluation of C(i), involves the attribution of the long-range weights $\phi$-specific to circular configurations- to the various ICC's $C_j(i)$ as defined in the exact treatment. With the logics of the linear scheme above, in order to cancel out this subdivision of C(i) into i-1 subclasses, we must merge the $C_k(i)$'s in a smooth-propagator $S_\phi[i]$, based on the appropriate expression of $\phi$ as a sum of J exponentials:

$$\varphi_{exp}(j) = \sum_{k=1}^{J} B_k \exp(\mu_k j) \qquad [5]$$

and the structure of $S_\phi[i]$ is a vector with J cells.

The processive evaluation of $S_\phi[i]$, involves, in turn, the processive handling of configurations with open base pairs at both extremities (left-ends relevant to $\phi$ and right-ends relevant to $\omega$). In order to treat the circular problem with algorithmic complexities relevant to the nearest-neigbour model (for the time complexity, for example, full reduction to O(n) and not only to the intermediate complexity $O(n^2)$), we need, for this category of configurations, to cancel out the third-order nested hierarchy of subdivsions associated with the definition of the ICC's $O_{k,j}(i)$, in the exact scheme (Fig.2c). As represented in Fig.3c, the "reverse" scheme -generalizing the cartoon in Fig.3a- leads in this case to a model of "nest of dolls", with the following fundamental structural and functional features:

1) A model of nest of dolls involves a hierarchy of levels, represented pictorially as "boxes" fitted one inside the other. This hierarchy in the nest of dolls substitutes for the nested hierarchy of subdivisions in the definition of the ICC's in the exact model. In both cases the hierarchy of sub-levels specifies increasingly more detailed configurational informations. The fundamental difference is that in the model of nest of dolls - generalizing the reasoning above for the linear model- each level corresponds to the merging of the enclosed sub-levels, associated with a numerical factoring, and the benefit of one order of magnitude reduction in algorithmic complexities.

In the circular model, the hierarchy in the nest of dolls involves an "intermediate" level between the uppermost level, standing for the single class O(i) of the nearest-neighbour treatment, and the lowest level represented by the ICC's of the model (with an appropriate treatment of the attached long-range informations which needs to be specified below).

Complexity build-up in the models -with an increasing number of mutually coupled long-range effects- is associated with models of nest of dolls involving an increasing number of such intermediate levels. In order to devise tractable formulations for complexity build-up, it is thus necessary to grasp the fundamental features in the handling of the information within such multi-levelled, hierarchical, merging schemes. It is also clear, as an afterthought, that such generality features could not be perceived properly in the "one-dimensional", linear, problem which lacks -by construction- any such intermediate level.

2) By essence, in a model of nest of dolls, there are several possible paths (following the number of intermediate levels involved) for defining the hierarchy in the merging operations.

In the circular problem, with one intemediate level, there are two such possible paths, as represented in Fig.3c. At the intermediate level, the merging of the ICC's could be performed either following the right-end or the left-end long-range informations (numerical factoring according to the lengthes of right-end open regions, or vice-versa).

For mutually coupled effects the -simultaneous- preservation of the integrity of the various long-range informations is required precisely because of the necessity of proceeding following the various possible paths. Following this requirement, as a difference from a static picture, a modularity must be introduced in the hierarchical merging scheme as described above: in order to treat *all* the coupled long-range effects with minimal algorithmic complexities, it must be possible -at each step- to reorganize the internal structure of the nest of dolls for implementing, for each effect, the corresponding appropriate hierarchy of numerical factorings.

The fundamental concepts in the detailed numerical implementation of such a model of nest of dolls, with modular merging hierarchy, can be illustrated with the circular problem.

For this model, the internal structure of the nest of dolls (as represented in Fig.3c) is first dictated by the processing of the right-end information ($\omega$-initiations and $\omega$-elongations). From this right-end viewpoint of the problem, the single class at the uppermost level of the hierarchy -standing for the O(i) of the nearest-neighbour model- is required to behave exactly as the smooth-propagator $S_\omega[i]$, above, for the linear model. Following this constraint, the basic merging scheme should -at this level- bear a formal analogy with equation [4], with the $S_\phi[j]$'s substituted for the $S_j$'s. In this analogy, it is the requirement for a full reduction of algorithmic complexities to a nearest neighbour formulation, which precludes the individual merging of the j-1 ICC's $O_{k,j}(i)$, following the linear scheme. Instead, in strict correspondence with one intermediate level box in the model of nest of dolls above, we need, numerically, to treat, this class of ICC's as a whole.

The smooth-propagator $S_\phi[j]$ (as evaluated as step j, through the merging of the j-1 ICC's $C_k(i)$, with $2 \leq k \leq j$) provides precisely the appropriate numerical representation - up to position j- for the merging of the j-1 $O_{k,j}$-(i)'s. For the -higher-order- merging of this class of ICC's, as a whole, we need accordingly to convert to the appropriate numerical representation the right-end information corresponding to $\omega$, common to all the members of the class (open base pairs from j+1 to i), with the left-end informations corresponding to $\phi$ - characteristic of each individual member of the class-already converted to the multiexponential numerical representations through the -first-order-merging in $S_\phi[j]$.

In this model of hierarchical mergings, with exactly the same reasoning as in the linear case, we are led to the requirement for a sliced treatment of the right-end long-range effect, following the multiexponential expression [3] for $\omega$. The critical consideration for the higher-order merging operation being now, at the step of $\omega$-initiation, the "attachment" of the I exponential handles to the smooth-propagator $S_\phi[i-1]$. In this operation it is the simultaneous preservation of the informational integrity of the mutually coupled effects $\omega$ and $\phi$ which is at stake. With this respect, at step i, following the second important consideration in the model of nest of dolls above, the requirement for the modularity in the merging hierarchy forbids, in the $\omega$-initiation step, any numerical factoring for the cells of $S_\phi[i-1]$, following the exponential components associated with $\omega$. Any such factoring, which could be written as $(S_{\phi(m)}[i-1] + S_{\phi(n)}[i-1])A_k \exp(4\lambda_k)$ (for two cells with indexes m and n, following the kth exponential component for $\omega$), would impede, subsequently, the numerical factorings following the mth and nth exponential components associated with $\phi$, and, accordingly, the desired reorganization in the merging hierarchy. The I exponential handles associated to $\omega$, must be attached, accordingly, to each cell $S_{\phi(m)}[i-1]$ of $S_\phi[i-1]$, taken individually (treated, with this respect, as an ICC of the linear model).

The higher order merging, can thus be represented -at this $\omega$-initiation step- as the product of the column-vector $S_\phi[i-1]$ with the row-vector $(A_1 \exp(4\lambda_1),..., A_I \exp(4\lambda_I))$ ($\omega$-initiation with one open base pair). The structure of the resulting JxI matrix, is representative -through the various iterative elongation steps-of the general numerical structure associated with the model of nest of dolls, and we call it the two-dimensional smooth-propagator $S_{\phi,\omega}[i]$ of the model.

As detailed in Fig.3d, through the above initiation step, the numerical contribution of an ICC $O_{k,i-1}(i)$ in $S_{\omega,\phi}[i]$ is represented in the cell with row index m and column index n ($S_{\phi(m),\omega(n)}[i]$), as $B_m \exp((2k-2)\mu_m)$-$S_{k,i-1}A_n \exp(4\lambda_n)$. By virtue of the numerical factoring in the representation of the right-end information, the i-2 ICC's $O_{k,i-1}(i)$ ($2 \leq k \leq i-1$) are represented, in the same cell, as $S_{\phi(m)}[i-1]A_n \exp(4\lambda_n)$ (corresponding to the intermediate level box representation in the model of nest of dolls).

More generally, with k coupled long-range effects, the rationale in this formulation is two-fold: 1) in each cell of the k-dimensional smooth propagator (with IxJx..L cells following the multiexponential expressions for the various long-range effects), each ICC is represented with the long-range informations converted to exponential handles and 2) the smooth-propagator allows the book-keeping of the IxJxL different "pictures" of this sum following all the possible combinations for the indexes of the exponential handles (in the cell with indexes i,j,..,1 the first long-range effect is expressed relative to the ith exponential component, the second effect is expressed relative to the jth exponential component, etc). This exhautive book-keeping of the various possible numerical representations of the merging scheme -following each exponential component of the multiexponential representations of all the long-range effects- allows independent manipulations of the coupled effects, and the desired reorganizations in the hierarchy as mentioned above. This feature is illustrated in the complete numerical treatment of the circular model -as reduced to a nearest-neighbour formulation- following the detailed implementation in Fig.3e.

In this implementation, in the prolongation of the linear model, the processive evaluation of $S_{\phi,\omega}[i]$ splits into IxJ calculations (not dependent on the length n of the sequence, and associated with the IxJ pictures of the merging scheme). In each calculation, in addition to the initiation contribution as detailed above, the right-end elongation contribution -following the nth exponential component associated with $\omega$- for the merged ICC's -following the mth exponential component associated with $\phi$- is performed in a single multiplicative step:

$$S_{\phi(m),\omega(n)}[i] = S_{\phi(m),\omega(n)}[i-1]\exp(2\lambda_n) + S_{\phi(m)}[i-1]A_n \exp(4\lambda_n) \qquad [6]$$

and the iterative application of this relation, leads, at step i, to the IxJ pictures for the hierarchical merging of the ICC's $O_{k,j}(i)$:

$$S_{\phi(m),\omega(n)}[i] = S_{\phi(m)}[2]A_n \exp(\lambda_n(2i-2)) + ... + S_{\phi(m)}[i-1]A_n \exp(4\lambda_n) \qquad [7]$$

which substitute to the I pictures (following the exponential components in $\omega$, alone) associated with equation [4], in the linear model.

Upon $\omega$-closures, by virtue of this book-keeping of the JxI pictures corresponding to the merging hierarchy, as first dictated by the right-end viewpoint of the problem, the desired reorganization in this hierarchy -following the left-end information $\phi$- is performed through a simple manipulation, based on a hierarchy in the reconstitutions of the multiexponential expressions $\omega_{exp}$ and $\omega_{exp}$. The addition -term by term- of the columns of $S_{\phi,\omega}[i-1]$, leads to the one-dimensional smooth-propagator designated as $S_{\phi,\omega}^-[i-1]$, associated with the contribution to $S\phi[i]$ of the configurations with an open base pair at position i-1 (after multiplication with $s_i$, for the weight of a closed base pair at position i). In this operation, upon the reconstitution of the multiexponential expression of the $\omega$'s (as the sums $\omega_{exp}$), the Ith cell of $S_{\phi,\omega}^-[i-1]$ writes:

$$S_{\varphi(m)\,\overline{\omega}}[i-1] = \sum_{n=1}^{I} S_{\varphi(m)\omega(n)}[i-1]$$

$$= \left[B_m \exp(4\mu_m)S_{2,2}\right]\omega_{exp}(2i-4) + .. + \left[\sum_{k=2}^{j} B_m \exp((2k)\mu_m)S_{k,j}\right]\omega_{exp}(2(i-j))$$

$$+ ... + \left[\sum_{k=2}^{i-2} B_m \exp((2k)\mu_m)S_{k,i-2}\right]\omega_{exp}(4)$$

$$= \sum_{k=2}^{i-2} B_m \exp((2k)\mu_m)\left[\sum_{j=k}^{i-2} S_{kj}\omega_{exp}(2(i-j))\right]$$

with the numerical factorings following the left-end informations ($B_1 \exp(4\mu_l)$, $B_1 \exp(6\mu_l)$, etc) as associated with the model of nest of dolls with the merging hierarchy following the left-end viewpoint of the problem, for the subsequent $\phi$-elongation and $\phi$-closure steps with the logics of a one-dimensional problem, and the benefit of numerical factorings following this effect as well (further detailed in Fig.3e).

## Non-exhaustive enumeration of some extensions and generalizations to the general procedure as described in the Patent:

### 1) Simultaneous handling of various long-range effects which are not mutually coupled:

As a difference to the case of mutually coupled effects, as described above, in such cases we consider the simultaneous treatment of various appropriate one-dimensional Smooth-Propagators, instead of Smooth-Propagators of higher dimensionalities.

### 2) Simultaneous handling of long-range effects with effects of specified ranges:

Typically we may consider the binding of an n-mer to DNA molecules. The n-mer "covers" n contiguous base pairs. If we designate by x the corresponding binding constant, for a processive formulation along the sequence, we shall slice, the attribution of such a weight into n local contributions $x^{1/n}$. If we consider a model in which such an effect is in competition with a long-range effect as defined in the helix-coil model (binding of n-mers to coiled regions), then the processive treatment of the model with reduced algorithmic complexities will involve a two-dimensional Smooth-Propagators (multi-dimensional in the most general case) which is represented as a nxl matrix, with l corresponding to the multiexponential representation of the long-range effect, and n associated to the sliced treatment of the effect whose range is specified to be n.

*3) Smooth-Propagators expressed in the complex domain:*

For models such as helix-coil transitions in DNA molecules with the topological constraint of supercoling, we must consider long-range effects (such as those associated with the energetic representation of global topological constraint as depending on the total fraction of open base pairs in the molecule) which are not monotonically decreasing. In such cases the appropriate representation of such long-range effects is with general complex exponentials (with real and imaginary parts), and correspondingly the appropriate formulation of Smooth-Propagators in the general complex domain.

*4) Hybrid formulations combining the exact representations of long-range effects up to a specified range, with multiexponential representations beyond this range:*

In the procedure of this invention it is easy to define such hybrid treatments.

5) *Application of the procedure of this invention to problems such as the prediction of secondary structures in RNA molecules.*

Such procedures rely on dynamic programming techniques for the evaluation of partition functions, and as a difference to the helix-coil transition model in DNA molecules we must also consider effects such as multiple loops, and define correspondingly the appropriate multiexponential representations.

*6) Multivariable Smooth-Propagators, based on multivariable multiexponential representations:*

Problems may be encountered where we need to represent as multiexponential functions, functions of several variables. The appropriate procedure is then the generalization of the

procedure as based on multidimensional multivariable smooth-propagators, based on such multivariable multiexponential representations.

Typical examples of potential applications concern models of helix-coil transitions in DNA molecules in competition with B-Z transitions. Another example of potential application is the prediction of beta-sheets in peptides and proteins, with the two variables in the function concerning the lengthes (in the course of a processive treatment) of the last strand (the one processively treated at step i) and that of the previous strand.

*7) Time-dependent treatments for large-scale models in the time and space domains:*

Methods such as molecular dynamics are essentially limited to time scales which will never reach the second. Simulation with such techniques of time-dependent properties for a small protein for one second, would require thousands years of computation time. In the prolongation of the procedure as presented in the Patent, time-dependent treatments can be developped -as inspired from Glauber's formulation in statistical phyiscs- which include in the same time long-range effects on the basis of the procedure presented here. Such formulations should represent the only possible appropriate solution for overcoming intrinsic limitations in methods such as molecular dynamics.

# <u>Legends of Figures 2 and 3:</u>

**Fig.2 Exact formulations in $O(n^k)$. Definition of the Irreducible Configurational Classes of a model through a nested hierarchy of subdivisions.**

We consider the derivation of exact formulations, the complexities of which are reduced to the intrinsic complexities of the models considered. By definition, in such schemes, only the strictly necessary information is handled throughout the treatment, based on the grouping together of the configurations into Irreducible Configurational Classes (ICC's of the model, see text).

In the definition of the ICC's, for a given model, the information of which it is kept track corresponds to specified states (0 or 1) for certain elements at given positions along the subsequence of length i, at step i, of the processive treatment. At positions where the states of the elements do not need to be specified, both states 0 and 1 are taken into account, signified by the symbol X. An ICC with m symbols X thus corresponds exactly to the grouping together of $C_{2;m}$ different configurations, and the numerical value of the ICC corresponds to the sum of the $C_{2;m}$ associated statistical weights.

The simplest illustration for the rationale in this formulation is represented in $\underline{a}$ for the nearest-neighbour model, in which, at each step, the configurations are split into two ICC's, $C(i)$ and $\overline{O}(i)$, according to the state of the last element, either in the closed or open state. If we designate by $cc(i)$, $oc(i)$, $oo(i)$ and $co(i)$ the weights attributed for the element at position i to be in the open (respectively closed state) following an element in the open, respectively closed state, then the nearest-neighbour scheme simply writes:

12

$$\begin{pmatrix} C(i) \\ O(i) \end{pmatrix} = \begin{pmatrix} cc(i) & oc(i) \\ co(i) & oo(i) \end{pmatrix} \begin{pmatrix} C(i-1) \\ O(i-1) \end{pmatrix}$$

which is the classical formulation -as found in many textbooks- with $cc(i) = \beta s_i$, $oc(i) = s_i$, $oo(i) = co(i) = 1$, and the overall treatment is formulated as the product of n such 2x2 matrices.

For the linear model, at step i, configurations belonging to the class O(i) cannot be treated as a whole. The operation represented as a single multiplication, O(i-1)oc(i), in the nearest-neighbour treatment involves now the attribution of the long-range weights $\omega$, associated with the ring-closures. The information of which it is kept track in O(i-1) is not sufficient, as for performing a ring-closure we need to know precisely the length of the right-end open region, and, accordingly, the position j of the last base pair in the closed state, for the subsequence of length i-1. For a given j we denote by $O_j(i)$ the sub-class of O(i) corresponding to configurations with a closed base pair at position j and open base pairs from j + 1 to i. One such class (with j = 7, at step i = 10) is represented in $\underline{b}$, with two particular configurations. Such a class is an ICC of the model as it specifies the necessary and sufficient information -relative to ring-closures- for the processive treatment of the model.

This treatment can be illustrated with the example in $\underline{b}$, highlighting the fundamental structure of an ICC as associated with an informational content and a numerical value. The numerical value of $O_j(i)$, corresponds to the sum of the statistical weights of the configurations grouped together, and is calculated, at step j, as $C(j) = S_j = s_i\omega(4)s_3 s_4 \omega(4)s_6 s_7 + s_2 s_3 \omega(8)s_7 + ....$(for the two particular configurations represented). For a given value of j, throughout the processive treatment, and the stepwise increase of i along the sequence, the numerical value of $O_j(i)$ remains constant and equal to $S_j$ (as the statistical weight attached to single-stranded free-ends is one). Nevertheless, despite such numerical constantness, a class $O_j(i)$ cannot be merged with any other class $O_k(i)$, as their informational contents (the lengthes i-j and i-k, respectively, of the right-end open regions, which increase stepwise with i) must be converted, at each step, upon ring-closures, to the corresponding long-range numerical contributions ($\omega(2(i-j) + 2)$ and $\omega(2(i-k) + 2)$, repectively). By virtue of the numerical factoring associated with the definition of an ICC, the conversion of the long-range information to the corresponding numerical contribution is performed, at a given step, through a single multiplication, for the various configurations of the class: the ring closure for the configurations in $O_j(i)$ is performed -at step i + 1- as $S_j\omega(2(i-j) + 2)s_{i+1}$, (and at step i + 2 -for the configurations in $O_j(i + 1)$- as $S_j\omega(2-(i-j + 1) + 2)s_{i+2}$, etc).

Following this scheme, the number of ICC's for configurations ending with an open base pair increases stepwise in the course of the processive treatment, with, for the numerical values, from step i-1 to step i, $O_j(i) = O_j(i-1)$ for $1 \le j \le i-2$ and the additional ICC $O_{i-1}(i) = C(i-1)$. The book-keeping of the information associated with the -second-order- subdivision of O(i) into the i-1 $O_j(i)$'s, is represented in $\underline{c}$, leading to a scheme in space complexity O(n). This book-keeping provides the necessary and sufficient information for the evaluation of C(i) -with the ring-closures performed following the various $O_j(i-1)$'s- the natural expression of which is the scalar product:

$$C(i) = s_i \left[ \sum_{j=1}^{i-1} O_j(i-1)\omega(2(i-j-1)+2) + C(i-1) + 1 \right]$$

(in this expression, the two last terms account, respectively, for the stacking of a closed base pair at position i on a closed base pair at position i-1, and the occurrence of a first closed base pair at position i). The time complexity for the evaluation of C(i) is thus in O(i), and for the n steps along the sequence, the overall time complexity of the sheme is in $O(n^2)$.

For generality considerations, it is important to notice that the above scheme -for the book-keeping of the information concerning the long-range effect- does not depend on the particular definition of the ground-state energy in the model, and the attribution of the weight one to free-ends. In a general case, end-effect weights $\omega^*(j)$ could be readily attributed to right-ends, respectively left-ends, with j contiguous elements in a specified state (represented here as "0"), as long as no correlations are introduced between the two ends. Correlation between the two ends represent precisely the intrinsic difficulty in the treatment of the circular model, in which left-ends and right-ends cannot be handled separately.

The exact treatment of the formal constraint of circularity, as introduced in the above linear model, is represented in $\underline{d}$. With such complexity build-up, for the definition of the ICC's of the model, it becomes necessary to specify increasingly more detailed informations as represented by the nested hierarchy of subdivisions in the configurational classes.

For the attribution of the specific weights attached to the long-range effect $\phi$, associated with circularization, the configurations in C(i) can no more be treated as a whole. It is necessary to specify in detail the informations concerning the left-ends of the configurations. More precisely, the circular problem splits into two parts: configurations with the element at position 1 in the closed state, and those with the element at position 1 in the open state. The first case is a problem strictly anlogous to a whole linear problem and can be treated exactly as such. Only the problem relevant to configurations with an open base pair at position 1 is considered. In the definition of C(i) -for configurations relevant to that category- it is necessary to specify the lengthes of the left-end open regions, and accordingly, as represented in $\underline{d}$, C(i) is subdivided into the i-1 ICC's $C_k(i)$, characterized by the number, k-1, of contiguous open base pairs at the left-ends.

For each $C_k(i)$, as represented pictorially in $\underline{d}$ ring-closures associated with $\omega$ are performed with exactly the same rationale as for C(i) in the linear model above. The specification of the left-end information in $C_k(i)$ -for the subsequent $\phi$-closures- makes it necessary to pick up in each class $O_j(i)$ (with j≥k, for the consistency of the constraints) those configurations with k-1 left-end open base pairs, to which $\omega$-closures are applied. This constraint, in turn, makes it necessary to introduce in O(i) the third-order subdivision, as represented in $\underline{d}$, in which each $O_j(i)$ is subdivided into the ICC's $O_{k,j}(i)$ (with 2≤k≤j), based on the information concerning the lengthes (k-1) of the left-end open regions. With this book-keeping of the information for the coupled effects $\phi$ and $\omega$, the space complexity of the model is in $O(n^2)$. The evaluation of a given $C_k(n)$ involves 1) $\omega$ ring-closures strictly equivalent to a whole linear problem, of size n-k + 1, and 2) one $\phi$-closure in a single step. The evaluation of $C_k(n)$ is accordingly in time complexity in $O((n-k + 1)^2)$, and the time complexity of the overall procedure, for the evaluation of the n-2 $C_k(i)$'s (with 2≤k≤n) is in $O(n^3)$.

With exactly the same rationale, complexity build-up can be further carried on, for the introduction in this formal circular model of the global topological constraint of supercoiling. For the definition of the corresponding ICC's, the specification in this model of the information concerning the total number (irrespective of their detailed distributions) of open base pairs -for a given $O_{k,j}(i)$- leads to a fourth-order subdivision in O(i). The overall time and space complexities of the corresponding scheme are respectively in $O(n^4)$ and $O(n^3)$.

**Fig.3 Rigorous formulations in O(n). One and multi-dimensional Smooth-Propagators.**

1) The linear problem. One-dimensional smooth propagator.

The cancelling out of the subdivision of O(i) into the i-1 Irreducible Configurational Classes $O_j(i)$ of the linear model, is represented as a cartoon in $\underline{a}$, with the $O_j(i)$'s "enclosed" in a "box", $S_\omega[i]$, called the one-dimensional smooth-propagator of the scheme, standing for the single class O(i) of the nearest-neighbour model.

In order to implement this model as a nearest-neighbour treatment, the various ICC's merged in the smooth-propagator must be treated as a whole in the "$\omega$ elongation" and "$\omega$ termination" steps. To do so we need to introduce a slicing in the task of the conversion of the long-range information into it's appropriate numerical representation. As detailed in the text, the only possibility for this is to express the long-range effect $\omega$ as a multiexponential function.

Based on the expression of $\omega$ as the sum of I exponential components, the numerical implementation of the nearest-neighbour treatment for the linear problem is represented in $\underline{b}$ (for the sake of illustration, I is taken equal to three), in strict correspondence with informational description in $\underline{a}$. The natural structure of $S_\omega[i]$ is a vector with I cells, each cell being devoted to the calculations relative to one exponential component, preserving -following this component- the fundamental property attached to a single exponential (exp(x)exp(y) = exp(x + y), at the basis of the slicing mentioned above). The kth cell $S_{\omega(k)}[i]$ (with 1≤k≤I) of $S_\omega$-[i] reflects strictly the merging of the ICC's $O_j(i)$, with the various long-range informations converted to numerical contributions relative to the kth exponential component, called the kth exponential handle. With S $_j$designating the numerical content of $O_j(i)$ in the exact treatment (evaluated as C(j) at step j, see Fig.2), $S_{\omega(k)}[i]$ writes:

$$S_{\omega(k)}[i] = \sum_{j=1}^{i-1} S_j A_k \exp[(2(i-j)+2)\lambda_k]$$

With the splitting of the calculations into I such terms, a consistent scheme can be implemented for the sliced treatment of the long-range information following *initiation, elongation and termination* steps, with the linear problem handled as a nearest-neighbour model:

1) Evaluation of $S_{\omega}[i]$ (standing for O(i) in the nearest-neighbour model) as a vector with I cells involving the $\omega$-initiation (standing for C(i-1)co(i)) and $\omega$-elongation (standing for O(i-1)oo(i)) steps.

- In the elongation step, the long-range informations for the various ICC's merged in $S_{\omega}[i-1]$ are reactualized. The reactualization is performed by addition of the element at position i, in the open state, endowed with it's partial contribution to the global long-range weights. For the kth cell, by virtue of the numerical factoring allowed by the kth exponential handle, this elongation is performed as the single multiplication $S_{\omega(k)}[i-1]\exp(2\lambda_k)$. The local contribution corresponds to the adding of two elementary single-stranded links to open regions, and the exponential multiplication is performed without the prefactors $A_k$.

- In the initiation step, the ICC $O_{i-1}(i)$, with one open base pair at the right-end, is merged with the other ICC's in the -reactualized- smooth-propagator. For this merging, following the above discussion, the I exponential handles ($A_1 \exp(4\lambda_1),...,A_l \exp(4\lambda_l)$) are "attached" to the scalar C(i-1) = $S_{i-1}$, and the kth cell -$S_{i-1}A_k\exp(4\lambda_k)$- of the resulting I-demensional vector is added to $S_{\omega(k)}[i]$. In this open region initiation operation, it is taken into account of the four single-stranded elementary links corresponding to a first open base pair (with\l\SU( k = 1;l; $A_k\exp(4\lambda_k)$) = $\omega(4)$)), and the exponential multiplication is performed taking into account the prefactors $A_k$.

2) Evaluation of the scalar term C(i) associated with two contributions, only one of them involving a long-range information handling, the w-closure step (standing for O(i-1)oc(i)).

- In the $\omega$-termination step, the ring-closures with base pair at position i (statistical weight $s_i$) is performed for the various merged ICC's $O_j(i)$, as a whole, based on the the "reconstitution" of the associated long-range weights $\omega(2(i-j))$, following their multiexponential expressions. This reconstitution is performed upon summing the I cells of $S_{\omega}[i-1]$ ,as represented in **b**, with the arrow leading to the scalar term $\overline{S_{\omega}}[i-1]$:

$$s_i \overline{S_{\omega}}[i-1] = s_i \sum_{k=1}^{I} S_{\omega(k)}[i] = s_i \sum_{k=1}^{I} \sum_{j=1}^{i-1} S_j A_k \exp[(2(i-j)+2)\lambda_k]$$

$$= s_i \sum_{j=1}^{i-1} S_j (\sum_{k=1}^{I} A_k \exp[(2(i-j)+2)\lambda_k]) = \sum_{j=1}^{i-1} S_j \,\omega(2(i-j)+2)s_i$$

- The "stacking" of the base pair at position i, in the closed state, on configurations ending at position i-1 with a base pair in the closed state (grouped in the ICC C(i-1), is treated exactly as in the nearest-neighbour model (C(i-1)cc(i) = $s_i$C(i-1)).

## 2) The circular problem. Two-dimensional smooth propagator.

A mentioned above, the circular problem splits into two parts: configurations with base pair at position 1 of the linearized molecule in the closed state, and configurations with base pair at that position in the open state. The first case is strictly analogous to a whole linear problem (in time complexity $O(n^2)$), and it's treatment can be reduced to complexity $O(n)$ with exactly the same rationale than for the linear problem ($\omega$

expressed as a multiexponential function). For such configurations with a closed base pair at position 1, the scalar C(i) (associated with the subsequence of length i) once evaluated, the completion to circular configurations (open base pairs from i + 1 to n) is readily performed through mutiplication with the (scalar) weight $\phi(2(n-i) + 2)$.

We concentrate here on the second case, and in what follows we refer to C(i) as associated with configurations with an open base pair at position 1, a closed base pair at position i and open base pairs from i + 1 up to n, which corresponds to the genuine circular problem, involving correlations between the extremities of a linearized molecule.

As detailed in the text, and in correspondence with the cartoon representation in a for the linear model, the reduction of algorithmic complexities for the circular problem is based on a model of nest of dolls following the representation in c. The model of nest of dolls results from the requirement of cancelling-out the third-order subdivision associated with the specification of the ICC's of the model (Fig.2d), along with the critical constraint of the preservation of the informational integrity relative to the mutually coupled long-range effects $\omega$ and $\phi$.

In order to fulfill the above two constraints, it must be possible to introduce the appropriate modularity in the hierarchical merging scheme, at the basis of the model of nest of dolls, for the benefit of numerical factorings following the various long-range effects involved. In c, for the circular model, the cartoon representation of such a modular merging is illustrated with the two possible paths for the definition of the single configurational class standing for the O(i) of the nearest-neighbour treatment: numerical factorings following the right-end effect $\omega$ as represented by path 1, and, alternatively, numerical factorings following the left-end effect $\phi$ as represented by path 2. In the cartoon representation, relative to a configurational description of the problem, such a modularity can be achieved only by a proper "shuffling" of the ICC's -between the intermediate level "boxes" of the model of nest of dolls- for switching from the hiearchical merging associated with path 1 to that associated with path 2.

The numerical implementation of such a modular scheme of hierarchical merging is realized by the two-dimensional smooth propagator $S_{\phi,\omega}[i]$ of the model, allowing the handling of configurations with open base pairs at both extremities, left-ends relevant to $\phi$ and right-ends relevant to $\omega$. In strict correspondence with the hierarchy in the model of nest of dolls represented in c, for the handling of the right-end information, attached to $\omega$, $S_{\phi,\omega}[i]$, is iteratively constructed as the merging of the $S_{\phi}[j]$'s (instead of the C(j)'s in the linear model) for the benefice of the numerical facrtorings following the left-end information $\phi$. As for the linear model prior to any such merging, at the $\omega$-initiation step, an appropriate numerical representation for the right-end information $\omega$ must be adopted for the subsequent processive elongation of the scheme with a nearest-neighbour logics. This constraint leads to a multiexponential expression for $\omega$, but, as detailed in the text, the preservation of the informational integrities -for the subsequent treatments following the left-end effect $\phi$- imposes to attach the I exponential handles $\Omega(4) = (A_1 \exp(4\lambda_1),...., A_I\exp(4\lambda_I))$ corresponding to the initiation of an right-end open region with one open base pair, to each cell $S_{\phi(j)}[i-1]$ of $S_{\phi}[i-1]$, treated individually. As such, the overall initiation operation is represented as the product of the column vector $S_{\phi}[i-1]$, with J cells, with the row vector $\Omega(4)$, with I cells, leading for $S_{\phi,\omega}[i]$ to a JxI matrix structure. The effect of such an initiation operation is illustrated in d relative to one particular ICC, amongst the i-2 ICC's merged in $S_{\phi}[i-1]$, characterized by 3 open base pairs at the left-end. For the sake of illustration it is supposed that $\omega_{exp}$ is expressed -as for the linear case- as the sum of I = 3 exponential components, and $\phi_{exp}$ as the sum of J = 2 exponential components: $\phi_{exp}(j) = B_1 \exp(\mu_1 j) + B_2 \exp(\mu_2 j)$.

With such a JxI matrix structure for $S_{\phi,\omega}[i]$, a complete cycle of itertative calculations is illustrated in e, as implementing the nearest-neighbourt treatment for the circular model, and involving now two initiation, elongation and termination steps, respectively for $\omega$ and $\phi$:

- The first line of this processive treatment evaluates $S_{\phi,\omega}[i]$ with the contributions of $\omega$-initiations, as detailed above, and the $\omega$-elongations, represented as the product of $S_{\phi,\omega}[i-1]$ with the diagonal matrix the kth element of which is $\exp(4\lambda_k)$. Exactly as represented in the model of nest of dolls, this elongation is performed with the benefice of the hierarchical factoring with respect to the $\phi$-information, through a single multiplication with each one of the J cells ($1\leq j\leq J$) $S_{\phi(j),\omega(k)}[i]$ of $S_{\phi,\omega}[i]$, instead of the single cell $S_{\phi(k)}[i-1]$ in the linear model.

- As represented in the second line, $S_{\phi}[i]$, in turn, is the sum of three contributions representing 1) the stacking of a closed base pair at position i on a closed base pair at position i-1, simply calculated as $s_i S_{\phi}[i-1]$; 2) the initiation of the left-end open region $\phi$, with i-1 base pairs, to which, at this step, corresponds the single configuration characterized by open base pairs from 1 to i-1 and a closed base pair at position i. Concerning an initiation, this operation is performed taking into account the prefactors $B_j$, and we can notice that, as a difference from the treatment of $\omega$, the handling of $\phi$- through it's multiexponential expression- is sliced into only two parts, the open region initiated at this step being completed only at the step of $\phi$-elongations and closures, below. 3) The step of $\omega$-closure is ensured by adding term by term the l columns of $S_{\phi,\omega}[i-1]$, and multiplication with $s_i$ (for the closed base pair at position i), for the multiexponential reconstitution of the long-range weights relative to $\omega$, the resulting one-dimensional smooth propagator (with J cells) is designated as $S_{\phi,\omega}^{-}[i]$, and in strict correspondence with the reorganization of the merging hierarchy -as represented with the shuffling cartoon- it allows the factorings following the exponential components associated with $\phi$. With $S_{\phi,\omega}^{-}[i]$ the one-dimensional smooth-propagator with J cells, the lth cell of which is given by the above experrssion is now amenable for j-elongation and j-closure with the benefice of apporpriate numerical factorings.

With $S_{\phi,\omega}^{-}[i]$ the one-dimensional smooth-propagator with J cells, the lth cell of which is given by the above experrssion is now amenable for j-elongation and j-closure with the benefice of apporpriate numerical factorings.


# <u>Bibliography:</u>

1 Poland D. and Scheraga H.A., *Theory of the Helix Coil Transition in Biopolymers* (Academic Press, New York) 1970.

2 Flory P. J., *Statistical Mechanics of Chain Molecules* (Hanser Publishers, Munich Vienna New York) 1989.

3 Zimm, B.H. *J Chem. Phys.* **33**, 1349-1356 (1960).

4 Litan, A. *J. Chem. Phys.* **49**, 2294-2300 (1968).

5 Poland, D. *Biopolymers* **13**, 1859-1871 (1974).

6 Fixman, M. & Freire, J.J. *Biopolymers* **16**, 2693-2704 (1977).

7 Yeramian, E., Schaeffer, F., Caudron, B., Claverie, P. & Buc, H. *Biopolymers* **30**, 481-497 (1990).

8 Jacobson, H. & Stockmayer, W.H. *J Chem. Phys.* **18**, 1600- (1950).

9 Fisher, M.E. *J Chem. Phys.* **45**, 1465- (1966).

10 Schwarz, G. *Biopolymers* **14**, 1173-1179 (1975).

11 Schwarz, G. & Schrader, U. *Biopolymers* **14**, 1181-1195 (1975).

12 Laiken, N. *Biopolymers* **12**, 11-26 (1973).

13 Anshelevich, V.V., Vologodskii, A.V., Lukashin, A.V. & Frank-Kamenetskii, M.D. *Biopolymers* **18** , 2733-2744 (1979).

14 Benham, C.J. *J. Chem. Phys.* **72**, 3633-3639 (1980).

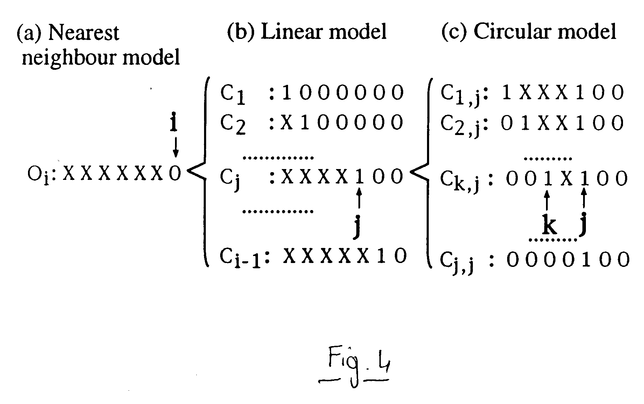

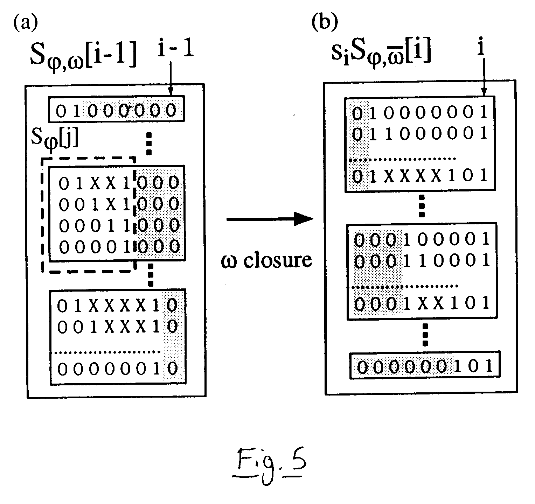

A further description of the present invention is given herebelow in detail for the case of K, mutually coupled, long-range effects. Reference is made to figures 4 and 5.

A general class of one-dimensional, sequence-specific Ising models is defined, with length-dependent long-range effects extending throughout the lengths, n, of the systems. The models are relevant to helix-coil transitions in DNA molecules. With k, *mutually coupled*, long-range effects the models provide a simple prototype for intractable problems, with algorithmic time complexities growing in $O(n^{k+1})$. It is shown that there is a single rigorous solution which reduces the complexities of the models from $O(n^{k+1})$ to $O(n)$, with the long-range effects expressed as multiexponential functions. Using this method, partition functions may be evaluated much faster than with an exact treatment. The calculations are performed with prescribed numerical accuracy, following the accuracy of the multiexponential representations of the long-range effects. For relevant classical models (with $k = 2$ or 3, and $n = 5000$), the calculation times are reduced by factors ranging between thousands and millions, with accuracy better than 1%.

We consider the evaluation in statistical mechanics of partition functions associated with models of biological macromolecules. Realistic formulations must take account of both sequence-specificity *and* various long-range effects. With an increasing number of, mutually coupled, long-range effects, such requirements lead most often to intractable algorithmic treatments, with calculation times prohibitive on any computer. Usually the only solution is to simplify considerably the physics of the interactions in the models. We have searched for alternative solutions, in which tractability in the algorithms is not obtained at the price of oversimplifications in the models.

Because of the formidable theoretical difficulties involved, it is not possible to consider such a problem in the most general case. Rather, for theoretical and practical reasons, we consider relatively simple model-problems which nevertheless present the intractability features in many structural models of interest. More precisely, we consider the introduction in the texbook classics model of helix-oil transitions in linear DNA (- [1]) of, mutually coupled, long-range effects in increasing number. For this class of model-problems, it is shown that there is one, and only one, solution that is rigorous and tractable. With this solution, calculation times may be reduced to orders relevant to the, simplest, nearest-neighbour models. The only approximation in the formulations concerns the numerical expression of the various long-range effects as multiexponential functions. Appropriate methods in signal analysis (the Padé-Laplace method [2]), allow control of the accuracy of such multiexponential representations, so that statistical mechanics calculations may be performed with prescribed numerical accuracy.

The general principles presented, and the conclusion that there is a single tractable, rigorous solution, are relevant for other macromolecular problems, such as the secondary structures of RNAs and peptides.

We shall consider a simple model of a DNA molecule, comprising n elements, or base pairs, which may exist in two states. The coiled state is represented by 0, for open, and the helical state is represented by 1, for closed. According to the sequence, a weight $s_i$ (equilibrium constant) is attributed to a base pair in the helical state, at position i, depending on its chemical composition: $s_{AT}$ for AT base pairs (two hydrogen bonds), or $s_{GC}$ for G-C base pairs (three bonds). Because of the sequence-specificity, partition functions must be evaluated sequentially along the molecule, using dynamic programming techniques [3]. From the partition functions, various quantities of interest (probability for an element to be in the open state, etc) are readily evaluated.

Algorithmic complexity "measures" the difficulty in calculating partition functions, dependent on the number and representation of long-range effects. Algorithmic time and space complexities in $O(g(n))$ underly calculation times and memory storage requirements which grow as $g(n)$.

Long-range effects are considered with respect to coiled states. In a nearest-neighbour model, a constant weight ($\sigma$) is attributed to a "loop" of j contiguous open base pairs, with the ground-state corresponding to open base pairs at the ends of a linear molecule (unconstrained single-stranded regions, with the weight 1). In the classical linear model, a length-dependent weight $\omega(2j+2)$, is attributed to such a loop. As evaluated in polymer physics [4], this loop-entropy weight reflects the closure probability for a single-stranded chain with $2j+2$ elementary links. We consider here additional long-range effects which are coupled with $\omega$. We shall say that two effects are mutually coupled, if each representation of one effect necessitates a full combinatorial description of the other one, compatible with the first effect (see below).

The constraint of circularity, for helix-coil transitions, is one such effect: single-stranded free-ends in the linear configuration, of lengths j and k respectively, are fused together, leading to a loop of length $j+k$. A loop-entropy weight, which we label $\phi(2(j+k)+2)$, must be attributed to such an interior coiled region. The physical representation of this effect is identical to that of $\omega$. Thus in a "formal" circular model which involves both effects $\omega$ and $\phi$, the statistical weight attributed to a sample-configuration such as 001101000110 is written $(s_3 s_4 \omega(4) s_6 \omega(8) s_{10} s_{11}) \phi(8)$, with the term between parentheses corresponding to the linear configuration alone. Another length-dependent long-range effect, the topological constraint of supercoiling can also be introduced into this circular model [5]. In such a scheme, with k mutually coupled long-range effects, the algorithmic time complexity grows as $O(n^{k+1})$.

For the classical linear problem, the Poland-Fixman-Freire [6] and the F__SIMM [7] formulations reduce the time complexity from $O(n^2)$ to $O(n)$. These formulations result in a reduction of only one order of magnitude for models in $O(n^{k+1})$. Here we wish to reduce instead complexities from $O(n^{k+1})$ to $O(n)$. The concepts are developed and illustrated with the reduction of the time complexity of the formal circular model, from $O(n^3)$ to $O(n)$. The principles can be readily extended to more complex models, with additional long-range effects.

For a given sequence, and a given model, we shall evaluate partition functions by processing sequentially from position 1 up to position n. We consider only configurations with at least one element in the closed state. The partition functions split into n terms C(i), the partition functions corresponding to configurations with a closed base pair at position i and open base pairs from $i+1$ to n.

For the nearest-neighbour model, at step i, we need only to retain information on the state of the element at position i-1. The evaluation of the partition function splits into the evaluation of $C_i$ and $O_i$, the partition functions (for the sub-sequence of length i) associated with configurations with a closed, respectively, open base pairs at position i. The recurrency relations are written:

$$C(i) = C_i = C_{i-1}cc(i) + O_{i-1}oc(i) \text{ and } O_i = O_{i-1}oo(i) + C_{i-1}co(i) \qquad (1)$$

with $cc(i) = s_i$, $oc(i) = \sigma s_i$, and $oo(i) = co(i) = 1$ the weights attributed for the element at position i to be in the closed (c), respectively open (o) states, following the state of the element at position i-1.

For the linear model, for $\omega$ ring-closures, we need to know the lengths of the right-end open regions. As represented in Fig.1, $O_i$ splits into the i-1 $C_j$'s (with $1 \leq j \leq i-1$), which must be retained. The evaluation of C(i) is written as a scalar product [7], the size of which increases stepwise:

$$C(i) = C_i = \sum_{j=1}^{i-2} C_j \, \omega(2(i-j)) s_i + C_{i-1} s_i \tag{2}$$

In order to attribute weights $\phi$, in a circular model, we must also know the lengths of the left-end open regions. $C(i)$ splits into the $i$ $C_{j,i}$'s $(1 \leq j \leq i)$, the partition functions, for the sub-sequences from $j$ to $i$, associated with configurations with closed base pairs at positions $j$ and $i$. $C(i)$ is written:

$$C(i) = \sum_{j=1}^{i} C_{ji} \, \varphi(2(n-i+j)) \tag{3}$$

The evaluation of each $C_{j,i}$ is a linear problem of length $i-j+1$, and so the $i-j$ $C_{j,k}$'s (with $j \leq k \leq i-1$) must be retained for right end $\omega$-closures, as represented in Fig. 4. The computation is in time complexity $O(n^3)$.

To reduce the algorithmic complexities of the linear and the circular models, we must cancel out the nested hierarchy of subdivisions, associated with the configuration classes as shown in Fig.4.

For the linear model, we want to treat the i-1 $C_j$'s as a whole, merging them in a single class, standing for $O_i$. We must devise a "sliced" treatment for the long-range effect, in which partial contributions add up, at each step, so that long-range effects $\omega(2(i-j))$ corresponding to each merged $C_j$ may be globally reconstituted. To reduce the algorithmic treatment of this model to that of the nearest-neighbour model (Eq.(1)), using a numerical factoring, the sliced treatment of the long-range effect must fulfill the condition:

$$O_i = \left[ \sum_{j=1}^{i-2} C_j \, \omega(2(i-j)) \right] oo(i) + C_{i-1} co(i) = O_{i-1} oo(i) + C_{i-1} co(i)$$

$$= \sum_{j=1}^{i-1} C_j \, \omega(2(i-j)+2) \tag{4}$$

The local, partial, contributions $co(i)$ and $oo(i)$ to $\omega$ do not depend on the position i. To fulfill Eq.(4) we must set $co(i) = \omega(4)$ (*initiation* of an open region with four elementary links) and $oo(i) = \omega(2)$ (*elongation* of open regions, with two additional elementary links), and require the condition $\omega(2(i-j))\omega(2) = \omega(2(i-j)+2)$ - [7]. The only mathematical solution for the condition $\omega(x)\omega(y) = \omega(x+y)$, is the exponential function $\omega_{exp}(x) = \exp(\lambda x)$ (with $\lambda$ a constant). There is no physical reason for $\omega$ to be of such a form. However $\omega$ may be represented numerically to a desired precision with a multiexponential expression [2]:

$$\omega_{exp}(j) = \sum_{p=1}^{I} A_p \exp(j\lambda_p) \tag{5}$$

The fundamental property $(\exp(x)\exp(y) = \exp(x+y))$ is not valid for this multiexponential function. Therefore evaluation of $O_i$ (Eq. (4)) must be split into I separate calculations each one following an exponential component of Eq. (5). This calculation defines a vector with I terms ("cells" in spreadsheet terminology), we call the smooth-propagator, $S_\omega[i]$ of the model. The pth cell, $S_{\omega(p)}[i]$, of $S_\omega[i]$ represents exactly the merging of the $C_j$'s, with the long-range effect numerically represented following the pth exponential component in Eq.(5):

$$S_{\omega(p)}[i] = \sum_{j=1}^{i-1} C_j A_p \exp[(2(i-j)+2)\lambda_p] \tag{6}$$

The "immersion" of the linear model in the nearest-neighbour model may then be written, with $S_\omega[i]$ substituting formally for $O_i$:

$$S_{\omega(p)}[i] = S_{\omega(p)}[i-1] \exp(2\lambda_p) + C_{i-1}A_p\exp(4\lambda_p) \ (1{\leq}p{\leq}I)$$

and

$$C_i = s_i S\overline{\phantom{x}}_\omega [i-1] + s_i C_{i-1} \tag{7}$$

$S\overline{\phantom{x}}_\omega [i-1]$ is a symbolic representation for the *termination* of the long-range effects, associated with $\omega$ ring-closures. The reconstitution of the weights $\omega_{exp}$ (following Eq. (5)), relative to the various merged $C_j$'s, is obtained by summing the I cells in $S\omega[i-1]$:

$$S\overline{\phantom{x}}_\omega [i-1] = \sum_{p=1}^{I} S_{\omega(p)}[i-1] = \sum_{j=1}^{i-2} C_j \,\omega_{exp}(2(i-j)) \tag{8}$$

For the circular model, the reduction of the time complexity from $O(n^3)$ to $O(n)$, requires the hierarchical cancelling out of the two levels of subdivisions, in Fig.4. For configurations with a closed base pair at position 1, the treatment is equivalent to a linear problem of size n. Henceforth we refer to the circular problem for configurations with at least one open base pair at the left-end. For such configurations, the cancelling out is represented diagramatically in Fig.5(a), with a "nest of dolls" structure.

At the lowest level, the j-1 $C_{k,j}$'s ($2{\leq}k{\leq}j$) must be merged in a smooth-propagator, $S_\phi[j]$, following the left-end effects $\phi$. Formally, for the generality of the treatments, we consider a multiexponential expression $\phi_{exp}$ of $\phi$, distinct from that of $\omega$. With $\phi_{exp}$ the sum of J exponentials (coefficients $B_m$ and $\mu_m$, $1{\leq}m{\leq}J$), $S_\phi[j]$ is written:

$$S_{\varphi(m)}[j] = \sum_{k=2}^{j} C_{kj} B_m\exp(2\mu_m) \quad 1{\leq}m{\leq}J \tag{9}$$

At the upper level, the $S_\phi[j]$'s must be further merged in a single configurational class, standing for $O_i$. In such a higher-order merging, the right-end information for each merged $S_\phi[j]$, i-j open base pairs, must be converted to the mutiexponential representation $\omega_{exp}$ of $\omega$, for its sliced treatment.

The hierarchical merging is performed for two nested levels of numerical factorings in the treatment of $\omega$. In contrast to the linear model, with $\omega$-closures, and the reconstitution of the various $\omega_{exp}$ expressions, we must retain the information on the left-end effects $\phi$, as expressed through the exponential components of $\phi_{exp}$. At this step, we treat the $\phi$-elongations (with open base pairs from $i+1$ to n), with numerical factoring following the multiexponential representation of $\phi$, and perform $\phi$-closures to reconstitute the various expressions $\phi_{exp}$. In Fig.5(b), the corresponding requirements are represented diagramatically with an internal reorganization of the structure of nest of dolls, and the merging hierarchy dictated by the effect $\phi$.

The only solution for fulfilling the above requirements is:

1) to merge the $S_{\phi(m)}[j]$'s in a smooth-propagator $S_{\phi(m),\omega}[i]$, for each index m of the multiexponential representation $\phi_{exp}$, separately. The merging is performed following Eq.(6) of the linear model, with the $S_{\phi(m)}[j]$'s substituted to the $C_j$'s. Following the representation $\omega_{exp}$, $S_{\phi(m),\omega}[i]$ is defined by p cells ($1{\leq}p{\leq}I$),

with the pth cell given by:

$$S_{\varphi(m),\omega(p)}[i] = \sum_{j=2}^{i-1} S_{\varphi(m)}[j] \ A_p\exp((2(i-j)+2)\lambda_p) \qquad (10)$$

and,

2) to retain the J different pictures for the merging of the $S_\phi[j]$'s, corresponding to the $S_{\phi(m),\omega}[i]$'s ($1\leq m\leq J$), for each exponential component in $\phi_{exp}$. Standing for $O_i$, we call the corresponding structure the two-dimensional smooth-propagator $S_{\phi,\omega}[i]$ of the model. $S_{\phi,\omega}[i]$ is defined by JxI cells, with the expression of the cell with row index m and column index p, $S_{\phi(m),\omega(p)}[i]$ given by (10).

Following this "book-keeping" of the information in the model, the $\omega$-initiation and $\omega$-elongation step is written:

$$S_{\phi(m),\omega(p)}[i] = S_{\phi(m),\omega(p)}[i-1]\exp(2\lambda_p) + S_{\phi(m)}A_p\exp(4\lambda_p) \ (1\leq m\leq J, 1\leq p\leq I) \qquad (11)$$

With $\omega$-closures, and a closed base pair at position i, detailed information on the right-end open regions is no longer needed. The reconstitution of the $\omega_{exp}$ expressions is performed by adding, term by term, the I columns of $S_{\phi,\omega}[i]$, leading to $S_{\phi,\overline{\omega}}[i]$ with a structure of a one-dimensional smooth-propagator. $S_{\phi,\overline{\omega}}[i]$ represents the contribution, in $S_\phi[i]$, of configurations with an open base pair at position i-1 and a closed base pair at position i. The mth cell is given by:

$$S_{\varphi(m),\overline{\omega}}[i]= \sum_{p=1}^{I} S_{\varphi(m),\omega(p)}[i] = \sum_{k=2}^{i-1} B_m\exp(2k\mu_m) \ [ \ \sum_{j=k}^{i-1} C_{kj} \ \omega_{exp}(2(i-j)+2)] \qquad (12)$$

with the numerical factorings written according to the left-end values for $\phi$, corresponding to the diagram of Fig.5(b). The iterative, self-consistent, calculation of $S_\phi[i]$ is then written:

$$S_{\varphi(m)}[i] = s_i S_{\varphi(m),\overline{\omega}}[i-1] + s_iS_{\varphi(m)}[i-1] + s_iB_m\exp(2i\mu_m) \qquad (1\leq m \leq J) \qquad (13)$$

with the two last terms in Eq.(13) corresponding, respectively, to the contributions of configurations with a closed element at i-1, and to the $\phi$-initiation step with open elements from 1 to i-1.

The evaluation of $C_o(i)$ (contribution to C(i) of configurations with at least one open base pair at the left-end) is then completed in a straight forward manner through $\phi$-elongations (open elements from i + 1 to n) and $\phi$-terminations as:

$$C(i) = \sum_{m=1}^{J} S_{\varphi(m)}[i] \exp((2(n-i)+2)\mu_m) = \sum_{j=2}^{i} C_{ji} \ \varphi_{exp}(2(n-(i-j))+2) \qquad (14)$$

corresponding to the exact calculation in Eq.(3), with the desired two orders of magnitude reductions in the algorithmic complexities.

The formulation developed here is based on the hierarchical merging of smooth-propagators of increasing dimensionalities and may be termed simulation with exponentials).

With such a formulation, the rigorous modelling of complex equilibria in DNA molecules becomes possible. Using the concepts developed here, calculations for the formal circular model (for molecules of size n = 5000), are accelerated by a factor of the order 2000, with numerical accuracy better than 0.04 % (with 14 exponential components for $\omega$ and $\phi$). Such results will be described elsewhere, with a detailed presentation of the treatment for supercoiled molecules (models in $O(n^4)$, with the algorithms formulated in the complex domain, for long-range effects which do not decay monotonically). Extensions to other models (RNAs, peptides) will also be presented elsewhere. The concepts developed here may also find applications for various lattice problems in other fields.

## FIG.1:

For the various models, symbolic representation of the configurational classes which must be retained, with an open base pair at position i. For the circular model, only the subdivision of one $C_j$ into $C_{k,j}$'s is shown. A symbol X specifies positions at which both states 0 and 1 are taken into account, thus a class with k such symbols corresponds to the grouping together of $2^k$ different configurations.

## FIG.2:

(a) Schematic configurational representation for the higher-order merging of the one-dimensional smooth-propagators $S_\phi[j]$'s into the two-dimensional smooth-propagator $S_{\phi,\omega}[i-1]$ (b) With $\omega$-closures, schematic representation for the configurational informations in $S_{\phi,\omega}^-[i]$. For $\phi$-elongations and $\phi$-closures, only the information on the left-end open regions is retained in this one-dimensional smooth-propagator.

## References

1 Poland D. and Scheraga H.A., *Theory of the Helix Coil Transition in Biopolymers* (Academic Press, New York) 1970.
2 Yeramian E. and Claverie P., *Nature* , **326** (1987) 169.
3 Bellman R. and Kaluba R., *J. SIAM*, **8** (1960) 582.
4 Jacobson H. and Stockmayer W.H., *J Chem. Phys.*, **18** (1950) 1600.
5 Vinograd J., Lebowitz J. and Watson R., *J. Mol. Biol.*, **33** (1968) 173.
6 Fixman M. and Freire J.J., *Biopolymers*, **16** (1977) 2693.
7 Yeramian E., Schaeffer F., Caudron B., Claverie P. and Buc H., *Biopolymers*, **30** (1990) 481.

**Claims**

1. A method for modelling a partition function in a sequence of elements having a length n̲, said partition function taking into account at least two, mutually coupled long-range effects, each element being capable of taking either of a discrete number, equal or greater than two, of accessible states, in particular two respectively open and closed states for the case of a macromolecular sequence, said partition function Z(n) being equal to the sum of n individual terms C(i) each corresponding to a partial contribution to the total partition and iteratively calculated in a processive treatment along the sequence, each term being equal to the sum of the statistical weights at each element of the sequence for all the possible configurations of said sequence, said method being implemented in a computer having a processor and a memory and being characterized in that it includes the following steps :
   (a) representing each long-range effect in the form of a sum of exponential terms,
   (b) storing into said memory an initial smooth propagator in the form of a N-dimension matrix of cells, N being equal to the number of long-range effects taken into account and each dimension of the matrix being associated with one long-range effect, the size of the matrix for each dimension being equal to the number of exponential terms of the associated long-range effect, each cell of said smooth propagator being representative of the merging of successive terms $O_{...,k,j}(i)$ corresponding to irreducible configurational classes of said possible configurations;

(c) for each subsequence of length i, updating the contents of said N-dimensional smooth propagator by merging the corresponding irreducible configurational classes into the corresponding cells of said smooth propagator, said updating step including the following sub-steps:

(i) defining a sub-smooth propagator having N-1 dimensions for each index of one dimension of the N-dimensional smooth propagator,

(ii) repeating sub-step (i) for N-1 dimensions, whereby each time a one-dimension sub-smooth-propagator is obtained,

(iii) multiplying the contents each cell of each one-dimension sub-smooth propagator for the previous sub-sequence i-1 with a corresponding exponential term of the long-range effect associated to said one dimension,

(iv) attaching to the previously calculated term of the partition function along said one dimension a multi-exponential handle each term of which corresponds to each exponential term of the associated long-range effect,

(v) adding together the results obtained at sub-steps (iii) and (iv) and placing the obtained values in the respective cells of said one-dimensional sub-smooth-propagator, and then adding together the contents of said respective cells, whereby the N-dimensional smooth propagator becomes a N-1-dimensional smooth propagator;

(vi) repeating sub-steps (i) to (v) for all the dimensions of the initial smooth propagator, whereby the final step (v) provides the term C(i) of the partition function for said sub-sequence of length i;

(d) repeating step (c) for all sub-sequences of the sequence, whereby all terms C(1) to C(n) of the partition function are obtained.

2. A method according to claim 1, characterized in that said initial smooth propagator has two dimensions associated respectively to a first long range effect corresponding to length dependent weights in an ADN molecule and to a second long range effect corresponding to loop-entropy weights in said molecule.

3. A system for modelling a partition function in a sequence of elements having a length n, said partition function taking into account at least two, mutually coupled long-range effects, each element being capable of taking either of a discrete number, equal or greater than two, of accessible states, in particular two respectively open and closed states for the case of a macromolecular sequence, said partition function Z(n) being equal to the sum of n individual terms C(i) each corresponding to a partial contribution to the total partition and iteratively calculated in a processive treatment along the sequence, each term being equal to the sum of the statistical weights at each element of the sequence for all the possible configurations of said sequence, said system comprising a processor and a memory and being characterized in that it further includes:

(a) means representing each long-range effect in the form of a sum of exponential terms,

(b) said memory storing an initial smooth propagator in the form of a N-dimension matrix of cells, N being equal to the number of long-range effects taken into account and each dimension of the matrix being associated with one long-range effect, the size of the matrix for each dimension being equal to the number of exponential terms of the associated long-range effect, each cell of said smooth propagator being representative of the merging of successive terms $O_{...,k,j}(i)$ corresponding to irreducible configurational classes of said possible configurations;

(c) means for updating, for each subsequence of length i, the contents of said N-dimensional smooth propagator by merging the corresponding irreducible configurational classes into the corresponding cells of said smooth propagator, said updating means comprising:

(i) means for defining a sub-smoothpropagator having N-1 dimensions for each index of one dimension of the N-dimensional smooth propagator,

(ii) said defining means being called upon for N-1 dimensions, whereby each time a one-dimension sub-smooth-propagator is obtained,

(iii) means for multiplying the contents each cell of each one-dimension sub-smooth propagator for the previous sub-sequence i-1 with a corresponding exponential term of the long-range effect associated to said one dimension,

(iv) means for attaching to the previously calculated term of the partition function along said one dimension a multi-exponential handle each term of which corresponds to each exponential term of the associated long-range effect,

(v) means for adding together the results obtained from said multiplying means and said attaching means and for placing the obtained values in the respective cells of said one-dimensional sub-smooth-propagator, and then for adding together the contents of said respective cells, whereby the N-dimensional smooth propagator becomes a N-1-dimensional smooth propagator;

(vi) said means (i) to (v) being called upon for all the dimensions of the initial smooth propagator, whereby the term C(i) of the partition function for said sub-sequence of length i is generated;

(d) said updating means being called upon for all sub-sequences of the sequence, whereby all terms C(1) to C(n) of the partition function are obtained.

4. A system according to claim 3, characterized in that said initial smooth propagator has two dimensions associated respectively to a first long range effect corresponding to length dependent weights in an ADN molecule and to a second long range effect corresponding to loop-entropy weights in said molecule.

| A | T | G | C | A | Å | C | T | A | A | C | G | T | C | G |

Fig 1a

0 1 1 1 0 0 1 1 1 1 0 1 1 0 0

j                                                                 k

Fig. 1b

$s_i$

$s_i\omega(6)$

i-1   i

i

Circularization

Fig. 1c

$\varphi$

j + k

$\omega$

$\omega$

i

XXXXXX 0  =  XXXXXX 0–0  +  XXXXXX 1–0

O(i)          O(i-1)          C(i-1)          Fig.2a

i-1            i-1

XXXXXX 1  =  XXXXXX 0–1  +  XXXXXX 1–1

C(i)          O(i-1)          C(i-1)

Fig.2b

Information

i

$O_j(i)$     XXXXXX 1 0 0 0

j

Numerical value: $S_j$

O(i): X X X X X X X 0 ⟶ O₁(i): 1 0 0 0 0 0 0 0

O₂(i): X 1 0 0 0 0 0 0

O_{l-2}(i): X X X X X 1 0 0

O_{l-1}(i): X X X X X X 1 0

C(i): X X X X X X X 1 = 1 0 0 0 0 0 0 1 + X 1 0 0 0 0 0 1 + ----- + X X X X X 1 0 1 + X X X X X X 1 1

O₁(i-1)          O₂(i-1)          O_{l-2}(i-1)          C(i-1)

Fig. 2c

Fig. 2d

Fig. 3a

EP 0 661 647 A1

EP 0 661 647 A1

$$S_\omega[i] = S_\omega[i-1] \begin{array}{|c|} \hline e^{2\lambda_1} \\ \hline e^{2\lambda_2} \\ \hline e^{2\lambda_3} \\ \hline \end{array} + C(i-1) \begin{array}{|c|} \hline A_1 e^{4\lambda_1} \\ \hline A_2 e^{4\lambda_2} \\ \hline A_3 e^{4\lambda_3} \\ \hline \end{array}$$

$$C(i) = s_i \; S_{\overline{\omega}}[i-1] + s_i \, C(i-1)$$

Fig. 3b

## Fig. 3c

Path 2 ← → Path 1

$S_{4,i}$

| 0 | 0 | 0 | 1 | X | X | X | 1 | | 0 | ← | $A_1 exp(4\lambda_1)$ | $A_2 exp(4\lambda_2)$ | $A_3 exp(4\lambda_3)$ |

$[B_1 exp(8\mu_1)]\ S_{4,i}$

$[B_2 exp(8\mu_2)]\ S_{4,i}$

| $[B_1 exp(8\mu_1)]\ S_{4,i}\ [A_1 exp(4\lambda_1)]$ | $[B_1 exp(8\mu_1)]\ S_{4,i}\ [A_2 exp(4\lambda_2)]$ | $[B_1 exp(8\mu_1)]\ S_{4,i}\ [A_3 exp(4\lambda_3)]$ |
|---|---|---|
| $[B_1 exp(8\mu_1)]\ S_{4,i}\ [A_2 exp(4\lambda_2)]$ | $[B_2 exp(8\mu_2)]\ S_{4,i}\ [A_2 exp(4\lambda_2)]$ | $[B_2 exp(8\mu_2)]\ S_{4,i}\ [A_3 exp(4\lambda_3)]$ |

Fig. 3d

EP 0 661 647 A1

$S_{\varphi,\omega}[i]$    $S_{\varphi,\omega}[i-1]$    $S_{\varphi}[i-1]$

| 0 | X X X X X | 0 |   =   | 0 | X X X X X | 0 |–| 0 |   +   | 0 | X X X X X | 1 |–| 0 |

$\varphi$     $\omega$

$\omega$ elongation     $\omega$ initiation

| $\varphi_1,\omega_1$ | $\varphi_1,\omega_2$ | $\varphi_1,\omega_3$ |
| $\varphi_2,\omega_1$ | $\varphi_2,\omega_2$ | $\varphi_2,\omega_3$ |

=

| $\varphi_1,\omega_1$ | $\varphi_1,\omega_2$ | $\varphi_1,\omega_3$ |
| $\varphi_2,\omega_1$ | $\varphi_2,\omega_2$ | $\varphi_2,\omega_3$ |

$\begin{pmatrix} e^{2\lambda_1} & 0 & 0 \\ 0 & e^{2\lambda_2} & 0 \\ 0 & 0 & e^{2\lambda_3} \end{pmatrix}$

+

| $\varphi_1$ |
| $\varphi_2$ |

| $A_1e^{4\lambda_1}$ | $A_2e^{4\lambda_2}$ | $A_3e^{4\lambda_3}$ |

---

$S_{\varphi}[i]$    $S_{\varphi,\omega}[i-1]$    $S_{\varphi}[i-1]$

| 0 | X X X X X | 1 |   =   | 0 | X X X X X | 0 |–| 1 |   +   | 0 0 0 0 0 0 |–| 1 |   +   | 0 | X X X X X | 1 |–| 1 |

$\varphi$

$\omega$ closure     $\varphi$ initiation

| $\varphi_1$ |
| $\varphi_2$ |

$=$ $s_i$

| $\varphi_1$ | |
| $\varphi_2$ | |

$+$ $s_i$

| $B_1e^{(2i-2)\mu_1}$ |
| $B_2e^{(2i-2)\mu_2}$ |

$+$ $s_i$

| $\varphi_1$ |
| $\varphi_2$ |

---

    i     n     $S_{\varphi}[i]$

| 0 | X X X X X | 1 0 0 0 0 0 0 |   =   | 0 | X X X X X | 1 |–| 0 0 0 0 0 |

$\varphi$     $\varphi$

$\varphi$ elongation and closure

$C(i)$

$=$

| $\varphi_1$ | $e^{[2(n-i)+2]\mu_1}$ |
| $\varphi_2$ | $e^{[2(n-i)+2]\mu_2}$ |

Fig. 3e

33

EP 0 661 647 A1

**(a) Nearest neighbour model**

**(b) Linear model**

**(c) Circular model**

$$O_i: X X X X X X 0 \begin{cases} C_1 \quad : 1\ 0\ 0\ 0\ 0\ 0\ 0 \\ C_2 \quad : X\ 1\ 0\ 0\ 0\ 0\ 0 \\ \cdots\cdots\cdots \\ C_j \quad : X\ X\ X\ X\ 1\ 0\ 0 \\ \cdots\cdots\cdots \\ C_{i-1}: X\ X\ X\ X\ X\ 1\ 0 \end{cases} \begin{cases} C_{1,j}: 1\ X\ X\ X\ 1\ 0\ 0 \\ C_{2,j}: 0\ 1\ X\ X\ 1\ 0\ 0 \\ \cdots\cdots \\ C_{k,j}: 0\ 0\ 1\ X\ 1\ 0\ 0 \\ \\ C_{j,j}: 0\ 0\ 0\ 0\ 1\ 0\ 0 \end{cases}$$

*Fig. 4*

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| T | EUROPHYSICS LETTERS vol. 25, no. 1 , 1 January 1994 page 49-55 E. YERAMIAN 'Complexity and tractability. Statistical mechanics of helix-coil transitions in circular DNA as a model-problem' * the whole document * --- | 1-4 | G06F19/00 |
| D,A | BIOPOLYMERS vol. 30 , 1990 pages 481 - 97 E. YERAMIAN, F. SCHAEFFER, B. CAUDRON, P. CLAVERIE ET H. BUC 'An optimal formulation of the matrix method in statistical mechanics of one-dimensional interacting units: efficient iterative algorithmic procedures' * the whole document * --- | 1-4 | |
| A | THE JOURNAL OF CHEMICAL PHYSICS vol. 92, no. 8 , 15 April 1990 pages 5127 - 35 C P WOODBURY 'Matrix polynomial extension of the sequence-generating function method for macromolecular binding' * the whole document * --- | 1-4 | **TECHNICAL FIELDS SEARCHED** (Int.Cl.6) G06F |
| D,A | THEORY OF HELIX-COIL TRANSITIONS IN BIOPOLYMERS 1970 pages 30 - 69 D POLAND ET AL 'Chapter 4 "theory for chains of any length: the matrix approach"' * the whole document * ----- | 1-4 | |

The present search report has been drawn up for all claims

| Place of search | Date of completion of the search | Examiner |
|---|---|---|
| THE HAGUE | 7 June 1994 | Granger, B |

EPO FORM 1503 03.82 (P04C01)